# EUROPEAN PATENT APPLICATION

(11) **EP 4 112 637 A1**
(43) Date of publication of application: **04.01.2023**
(21) Application number: 20922203.3
(22) Date of filing: 17.12.2020
(51) Int. Cl.: C07K 14/605, C07K 14/575, A61K 38/26, A61K 38/22, A61P 3/10, A61P 3/04

(54) **POLYPEPTIDE COMPOUND AND APPLICATION THEREOF IN PREVENTION OR TREATMENT OF DIABETES OR DIABETES COMPLICATION**

(30) Priority: 24.02.2020 CN 202010113888
(71) Applicant: Shenzhen Turier Biotech Co., Ltd., Guangdong 518000 (CN)
(72) Inventor: JIANG, Xianxing, Guangzhou, Guangdong 510006 (CN); ZHAO, Qian, Guangzhou, Guangdong 510006 (CN)
(74) Representative: SSM Sandmair
(86) International application number: PCT/CN2020/137071
(87) International publication number: WO 2021/169512

(57) **Abstract**

The present invention discloses polypeptide compounds and use thereof in the prevention or treatment of diabetes or diabetic complications, which shows good long-acting hypoglycemic effect and therapeutic effect of diabetic nephropathy; it also has the effect of high enzymatic stability, high biological activity, and no occurrence of adverse effects, and can be used in the preparation of medicaments for the treatment of hyperphagia, obesity and overweight, elevated cholesterol, diabetes and diabetic nephropathy.

## Description

### TECHNICAL FIELD OF THE INVENTION

The present invention belongs to the field of biochemical technology, and specifically relates to polypeptide compounds and use thereof in the prevention or treatment of diabetes or diabetic complications.

### BACKGROUND OF THE INVENTION

In recent years, the prevalence of diabetes has shown a rapid increase worldwide with the rapid economic development, changes in dietary structure and lifestyle, and the accelerated demographic ageing. According to statistics, nearly 9% of adults worldwide suffer from type 2 diabetes. And the International Diabetes Federation (IDF) estimates that the global prevalence of diabetes will increase from 415 million in 2015 to 642 million in 2040 (Unnikrishnan R, Pradeepa R, Joshi SR, Mohan V. Type 2 Diabetes: Demystifying the Global Epidemic. Epidemic. Diabetes, 2017. 66:1432-1442). Diabetes has become another important chronic non-communicable disease that seriously endangers human health after cardiovascular and cerebrovascular diseases and tumors.

Several surveys in recent years have shown that the control of diabetes is not optimistic in both developed countries in Europe and the United States and in developing countries such as China. One of the most serious consequences of diabetes is the development of diabetic vasculopathy, which is clinically manifested by microvascular and macrovascular complications. Diabetic nephropathy, a microvascular complication, is the most common cause of end-stage renal disease in developed countries.

Approximately 40% of patients with type 1 diabetes will develop diabetic nephropathy, and although relatively few cases of type 2 diabetic nephropathy progress to end stage renal disease, the rapid increase in the number of patients with type 2 diabetes means that the incidence of diabetic nephropathy will also increase dramatically in the absence of effective measures for diabetes management. Therefore, there is an urgent need for effective and long-acting glucose-lowering drugs for the treatment of diabetic nephropathy. Because there is considerable ethnic variability in the incidence of diabetic nephropathy, only about one-third of patients with type 1 diabetes will develop diabetic nephropathy and it is strongly associated with genetic factors. Although relatively few cases of type 2 diabetic nephropathy progress to end-stage renal disease, the rapid increase in the number of patients with type 2 diabetes means that the incidence of diabetic nephropathy will also increase dramatically in the absence of effective measures for diabetes management, and therefore there is an urgent need for effective and long-acting hypoglycemic agents for the treatment of diabetic nephropathy (Chen L, Magliano DJ, Zimmet PZ. The worldwide epidemiology of type 2 diabetes mellitus-present and future perspectives. (Nat Rev Endocrinol, 2011, 8(4):228- 236).

With the intensively research on diabetes and its treatment, novel targets of drug action for the treatment of type 2 diabetes continue to be identified. GLP-1 analogs (Meier JJ. GLP-1 Receptor Agonists for Individualized Treatment of Type-2 Diabetes Mellitus. Nat. Rev. Endocrinol, 2012. 8(12):728), which was marketed in 2005 and have different targets of action than previous drugs, offer novel therapeutic options for the treatment of diabetes. GLP-1 analogs that have been approved for the treatment of type 2 diabetes include exenatide, liraglutide and lixisenatide, as well as the subsequent generation of albiglutide and dulaglutide. In addition, Novo Nordisk's once-weekly long-acting GLP-1 analogue semaglutide has also been marketed for the treatment of type 2 diabetes.

Compared with homologous ligand single-target agonists, GLP-1R/GCGR dual agonists have synergistic effects of glucose-lowering, lipid-lowering and weight-lowering. In addition, for GLP-1R/GCGR dual-target agonists, the synergistic effect of GCGR and GLP-1 may enhance pancreatic β-cell function while potentially minimizing or preventing the progression of type 2 diabetes. These innovations increase the established effects of GLP-1 and the second activity may increase additional efficacy, e.g., weight reduction, cardiovascular benefit, and treatment of diabetic nephropathy.

For example, a glucagon analogue for the treatment of metabolic diseases is disclosed in Chinese patent CN201711194175.0, but it was found that the glucagon analoguehas a comparable effect for improving glucose tolerance as liraglutide, but less than semaglutide, and the glucagon analogue likewise failed to meet the requirements for clinical use.

In conclusion, despite the existence of many drugs for the treatment of diabetes, the treatment of type 2 diabetes and the associated complications of nephropathy remains a challenge, and curbing the global spread of diabetic nephropathy is destined to be a long-term and difficult task. The development of novel dual- or multi-target agonist peptide drugs is a major direction for the treatment of diabetes and its complications.

### SMMMARY OF THE INVENTION

The purpose of the present invention is to provide an novel polypeptide compounds. The present inventors have demonstrated that such polypeptide compounds have a longer half-life and insulinotropic activity without adverse event through a great number of experimental studies. The polypeptide compounds can be used for the prevention or treatment of diabetes and diabetic complications such as diabetic nephropathy.

Another purpose of the present invention is to provide a use of the novel polypeptide compounds described above, which can potentially be used as an novel generation of medicament for preventing or treating diabetes and diabetic complications such as diabetic nephropathy, and also can be used for reducing blood glucose or body weight.

To achieve the above purpose, the technical solution of the present invention is as follows.

The present invention provides a polypeptide compound comprising a parent peptide represented by the following amino acid sequence:
His-Xaa2-Gln-Gly- Thr⁵-Phe- Thr-Ser-Asp-Lys¹⁰-Ser-Lys- Tyr-Leu-X aa15¹⁵-Xaa16-Xaa17-Ala-Ala-Gln²⁰-Xaa21-Phe-Xaa23-Xaa24-Trp²⁵-Leu -Xaa27-Xaa28-Gly-Gly³⁰-Pro-Ser-Ser-Gly-Xaa35³⁵-Pro-Pro-Pro-Ser,
wherein,
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gln;
Xaa27 = Leu or Lys;
Xaa28 = Asp, Glu or Ala;
Xaa35 = Ala or Aib.

Preferably, in the amino acid sequence of the parent peptide, Xaa2 = Aib or D-Ser.

Preferably, in the amino acid sequence of the parent peptide, Xaa21 = Asp.

Preferably, a carboxyl terminal of the amino acid sequence of the parent peptide is unmodified or is modified by an amino group to form a -CONH₂ group.

Moreover, preferably, a side chain of Lys at position 10 or 12 in the amino acid sequence of the parent peptide is linked to a lipophilic substituent via a bridging group, the bridging group is one selected from (PEG)ₘ, (PEG)ₘ-γGlu, (PEG)ₘ-Asp, (Gly)ₓ-(Gly-Ser)_{y}-(Gly)_{z}-, (Gly)ₓ-(Gly-Ser)_{y}-(Gly)_{z}-γGlu and (Gly)ₓ-(Gly-Ser)_{y}-(Gly)_{z}-Asp. The linkage is: a side chain amino group of the Lys at position 10 or 12 forms an amide bond attached to the parent peptide with the carboxyl group of a glycine residue of the bridging group or a carboxyl group of the (PEG)ₘ terminal modification at one end of the bridging group, in addition, the lipophilic substituent is linked by its carboxyl group to the amino group of the bridging group at the other end thereof by forming an amide bond. The lipophilic substituent is CH₃(CH₂)ₙC(O)- or HOOC(CH₂)ₙC(O)- and its acyl group forms an amide bond with the amino group in the bridging group.

In the bridging group described herein, m is an integer of 2 to 10; x is an integer of 0 to 5; y is an integer of 1 to 5; z is an integer of 1 to 5; and in the lipophilic substituent described herein, n is an integer of 14 to 20.

Alternatively, the Lys at position 10 or 12 in the amino acid sequence of the parent peptide may be replaced with HomoLys, Orn, Dap or Dab.

Further preferably, in the amino acid sequence of the parent peptide:
Xaa2 = Aib or D-Ser.
Xaa15 = Glu;
Xaa16 = Aib;
Xaa17 = Lys;
Xaa21 = Asp;
Xaa23 = Val;
Xaa24 = Glu;
Xaa27 = Lys;
Xaa28 = Ala;
Xaa35 = Ala;
or,
Xaa2 = Aib or D-Ser;
Xaa15 = Asp;
Xaa16 = Glu;
Xaa17 = Arg;
Xaa21 = Asp;
Xaa23 = Iva;
Xaa24 = Gln;
Xaa27 = Leu;
Xaa28 = Asp or Glu;
Xaa35 = Ala or Aib.

According to a specific embodiment of the present invention, the amino acid sequence of the parent peptide is selected from SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15 and SEQ ID NO. 16.

According to a specific embodiment of the present invention, the Lys at position 10 or 12 in the amino acid sequence of the parent peptide, preferably the Lys at position 10, is linked to the lipophilic substituent to form any one of the following structures:

### Lys(PEG₂-PEG₂-γGlu-CO(CH₂)₁₂CO₂H);

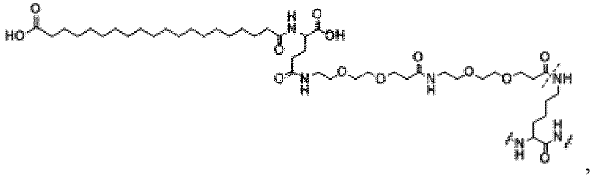

or

### Lys(PEG₂-PEG₂-γGlu-CO(CH₂)₁₃CH₃);

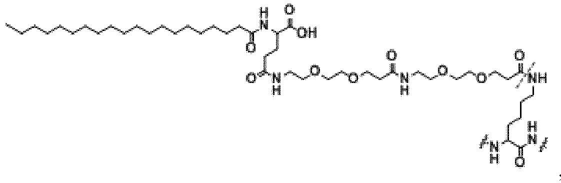

, or

### Lys(PEG₂-PEG₂-γGlu-CO(CH₂)₁₅CO₂H);

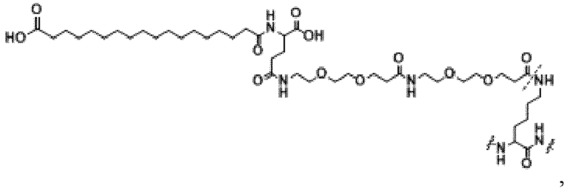

or

### Lys(PEG₂-PEG₂-CO(CH₂)₁₂CO₂H);

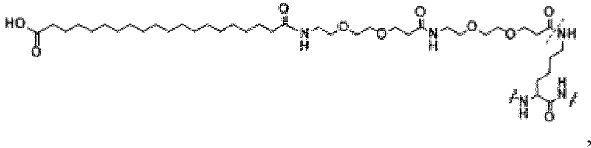

or

### Lys(Gly-Gly-Ser-Gly-Ser-Gly-γGlu-CO(CH₂)₁₈CO₂H)

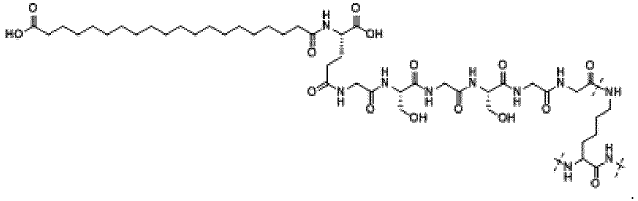

According to a specific embodiment of the present invention, the present invention provides any one of the following polypeptide compounds:
Compound 1:
   H-Aib-QGTFTSDK(PEG₂-PEG₂-γGlu-CO(CH₂)₁₈CO₂H)SKYLE-Ai b-KAAQDFVEWLKAGGPSSGAPPPS-NH₂
Compound 2:
   HsQGTFTSDK(PEG₂-PEG₂-CO(CH₂)₁₈CO₂H)SKYLE-Aib-KAAQ DFVEWLKAGGPSSGAPPPS-NH₂
Compound 3:
   HsQGTFTSDK(PEG₂-PEG₂-γGlu-CO(CH₂)₁₈CO₂H)SKYLE-Aib-K AAQDFVEWLKAGGPSSGAPPPS-NH₂
Compound 4:
   HsQGTFTSDK(PEG₂-PEG₂-γGlu-CO(CH₂)₁₆CO₂H)SKYLE-Aib-K AAQDFVEWLKAGGPSSGAPPPS-NH₂
Compound 5:
   HsQGTFTSDK(PEG₂-PEG₂-γGlu-CO(CH₂)₁₈CH₃)SKYLE-Aib-KA AQDFVEWLKAGGPSSGAPPPS-NH₂
Compound 6:
   HsQGTFTSDK(PEG₂-PEG₂-γGlu-CO(CH₂)₁₈CO₂H)SKYLDERAA QDF-Iva-QWLLDGGPSSG-Aib-PPPS-NH₂
Compound 7:
   HsQGTFTSDK(PEG₂-PEG₂-γGlu-CO(CH₂)₁₈CO₂H)SKYLDERAA QDF-Iva-QWLLEGGPSSGAPPPS-NH₂
Compound 8:
   H-Aib-QGTFTSDK(PEG₂-PEG₂-γGlu-CO(CH₂)₁₈CO₂H)SKYLDER AAQDF-Iva-QWLLDGGPSSG-Aib-PPPS-NH₂
Compound 9:
   H-Aib-QGTFTSDK(PEG₂-PEG₂-γGlu-CO(CH₂)₁₆CO₂H)SKYLDER AAQDF-Iva-QWLLDGGPSSG-Aib-PPPS-NH₂
Compound 10:
   H-Aib-QGTFTSDK(PEG₂-PEG₂-γGlu-CO(CH₂)₁₈CH₃)SKYLDERA AQDF-Iva-QWLLDGGPSSG-Aib-PPPS-NH₂
Compound 11:
   H-Aib-QGTFTSDK(PEG₂-PEG₂-γGlu-CO(CH₂)₁₈CH₃)SKYLDERA AQDF-Iva-QWLLDGGPSSG-Aib-PPPS-NH₂
Compound 12:
   HsQGTFTSDK(PEG₂-PEG₂-γGlu-CO(CH₂)₁₈CO₂H)SKYLDERAA QDF-Iva-QWLLDGGPSSG-Aib-PPPS-NH₂
Compound 13:
   HsQGTFTSDK(GGSGSG-γGlu-CO(CH₂)₁₈-COOH)SKYLE-Aib-K AAQDF VEWLKAGGPS SGAPPPS
Compound 14:
   HsQGTFTSDK(GGSGSG-γGlu-CO(CH₂)₁₈-COOH)SKYLE-Aib-K AAQDFVEWLKAGGPSSGAPPPS-NH₂
Compound 15:
   H-Aib-QGTFTSDK(GGSGSG-γGlu-CO(CH₂)₁₈-COOH)SKYLDER AAQDF-Iva-QWLLDGGPSSG-Aib-PPPS
Compound 16:
   H-Aib-QGTFTSDK(GGSGSG-γGlu-CO(CH₂)₁₈-COOH)SKYLDER AAQDF-Iva-QWLLDGGPSSG-Aib-PPPS-NH₂.

The present invention also provides a composition comprising a polypeptide compound as described herein.

Preferably, the composition is a pharmaceutical composition, which optionally further comprises a pharmaceutically acceptable carriers or excipients.

The present invention further provides a use of the polypeptide compound or the composition in the preparation of a medicament for preventing or treating diabetes and/or diabetic complications; wherein preferably, the diabetic complication is diabetic nephropathy.

The present invention further provides a use of the polypeptide compound or the composition in the preparation of a nutraceutical or medicament for reducing body weight.

The technical solution of the present invention is described in detail below:
In some embodiments, the present invention is to provide an novel polypeptide compound comprising a parent peptide represented by the following amino acid sequence:
His-Xaa2-Gln-Gly-Thr-Phe-Thr-Ser-Asp-Lys-Ser-Lys-Tyr-Leu-Xaa1 5-Xaa16-Xaa17-Ala-Ala-Gln-Xaa21-Phe-Xaa23-Xaa24-Trp-Leu-Xaa27-Xaa28- Gly-Gly-Pro-Ser-Ser-Gly- Xaa35-Pro-Pro-Pro-Ser-COR₁
wherein, R₁ = -NH₂ or -OH;
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gln;
Xaa27 = Leu or Lys;
Xaa28 = Asp, Glu or Ala;
Xaa35 = Ala or Aib.

In the amino acid sequence of the parent peptide, the side chain of Lys at position 10 or 12 is linked to a lipophilic substituent via a bridging group.

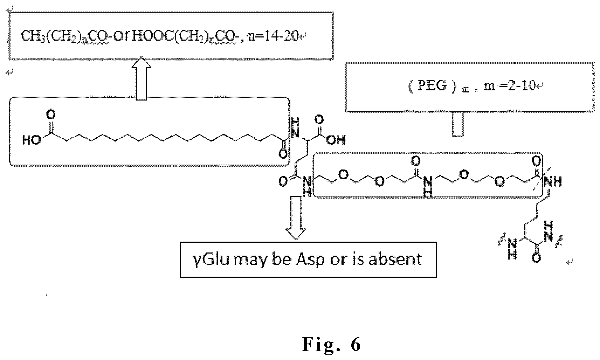

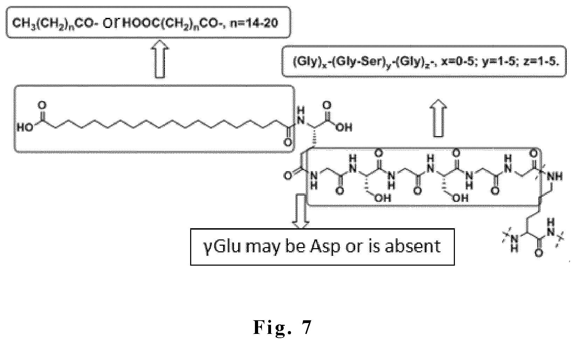

The bridging group comprises (PEG)ₘ, or (PEG)ₘ-γGlu or (PEG)ₘ-Asp; it may also comprise (Gly)ₓ-(Gly-Ser)_{y}-(Gly)_{z}-, or (Gly)ₓ-(Gly-Ser)_{y}-(Gly)_{z}-γGlu or (Gly)ₓ-(Gly-Ser)_{y}-(Gly)_{z}-Asp. The linkage is: the side chain amino group of the Lys at position 10 or 12 forms an amide bond attached to the parent peptide with the carboxyl group of the glycine residue or the carboxyl group of the (PEG)ₘ terminal modification at one end of the bridging group and attaches to the parent peptide; at the same time, the lipophilic substituent is linked by its carboxyl group to the amino group of the bridging group; in addition, the lipophilic substituent is attached to the amino group of the bridging group at the other end thereof by forming an amide bond with its carboxyl group. The Lys at position 10 or 12attached by the lipophilic substituent is capable of being replaced with HomoLys, Orn, Dap or Dab. wherein m is an integer of 2 to 10; x is an integer of 0 to 5; y is an integer of 1 to 5; and z is an integer of 1 to 5. The lipophilic substituent is selected from CH₃(CH₂)ₙCO- or HOOC(CH₂)ₙCO-, wherein n is an integer of 14-20, and the linkage is detailed in Fig. 6 and Fig. 7, Fig. 6 shows an exemplary modification of the Lys side chain in the parent peptide of the present compound, and Fig. 7 shows another exemplary modification of the Lys side chain in the parent peptide of the present compound.

As in the full specification of the present application, conventional three-letter codes are used to represent natural amino acids and accepted three-letter codes are used to represent other amino acids, such as Aib (aminoisobutyric acid), Orn (ornithine). The compounds of the present invention are based on theoretical intramolecular bridges that can stabilize the helical structure of the molecule, thereby improving potency and/or selectivity against the glucagon-like peptide 1 (GLP-1R) receptor and the glucagon (GCGR) receptor.

The polypeptide compounds of the present invention can be bound to albumin in the blood based on theoretical lipophilic substituents, thereby protecting the compounds of the present invention from enzymatic degradation and thereby increasing the half-life of the polypeptide compounds.

In some embodiments, the present invention is to provide pharmaceutical compositions containing the novel agonist polypeptide compounds of the present invention, made by adding pharmaceutically acceptable carriers and/or excipients with the novel agonist polypeptide compounds as active ingredients.

In some embodiments, the present invention is to provide pharmaceutical use of the novel agonist polypeptide compounds of the present invention. Cellular and animal experiments have shown that the novel agonist polypeptide compounds of the present invention have long-acting hypoglycemic effects and can be used as medicaments for the treatment of diabetes.

In some embodiments, the present invention is to provide other medicinal use of the novel agonist polypeptide compounds of the present invention. Cellular and animal experiments have shown that the novel agonist polypeptide compound of the present invention has a medicinal effect in the treatment of diabetic complications such as diabetic nephropathy. Animal experiments showed that the therapeutic effect was superior to that of the marketed GLP-1 analogue semaglutide, which can be used as a medicament for the treatment of diabetic complications such as diabetic nephropathy.

The novel agonist polypeptide compounds of the present invention can also reduce body weight and has potential use as a medicament for the treatment of obesity.

The parent peptide in the novel agonist polypeptide compounds mentioned in the present invention is a homologous polypeptide. Homologous peptide in the present invention means that the peptide originally has the amino acid sequence of gastrin regulator (OXM), glucagon-like peptide (GLP-1), exenatide, or glucagon, but one or more of the amino acid residues have been replaced by different amino acid residues that are conserved among themselves, and the resulting peptide can be used to implement the present invention.

The polypeptide compounds of the present invention can also be used to prevent weight gain or promote weight loss. The polypeptide compounds may cause a decrease in food intake and/or an increase in energy expenditure, resulting in an observable effect on body weight. Thus, the polypeptide compounds of the present invention may be used directly or indirectly to treat any condition caused by or characterized by excess weight, such as the treatment and/or prevention of obesity, morbid obesity, obesity-related inflammation, obesity-related gallbladder disease, and obesity-induced sleep apnea. The role of the polypeptide compounds of the present invention in these conditions may be due to or related to the action of the polypeptide compounds on body weight, or may be independent of their action on body weight.

It will be understood by those of skill in the art that the pharmaceutical compositions of the present invention are suitable for various modes of administration, such as oral administration, transdermal administration, intravenous administration, intramuscular administration, topical administration, transnasal administration, etc. Depending on the mode of administration used, the pharmaceutical compositions of the polypeptide compounds of the present invention can be made in various suitable dosage forms comprising at least an effective dose of the polypeptide compound of the present invention and at least a pharmaceutically acceptable pharmaceutical carrier. Examples of suitable dosage forms are tablets, capsules, sugar-coated tablets, granules, oral solutions and syrups, ointments and patches for skin surfaces, aerosols, nasal sprays, and sterile solutions that can be used for injection.

The pharmaceutical compositions containing the polypeptide compounds of the present invention can be made into solutions or lyophilized powders for parenteral administration, and the powders can be reconfigured by adding appropriate solvents or other pharmaceutical carriers prior to use, and the liquid formulations are generally buffers, isotonic solutions, and aqueous solutions.

The dosage of the pharmaceutical compositions of the present invention can be varied within a wide range and can be easily determined by the person skilled in the art based on objective factors such as the type of disease, severity of the disease, weight of the patient, dosage form, route of administration, etc.

The present invention has the following advantages when compared with the prior art:
1) The polypeptide compounds of the present invention have better biological activity when compared with natural OXM at the same mass number.
2) The polypeptide compounds of the present invention have better pharmacological effects for the treatment of diabetic complications such as diabetic nephropathy when compared with GLP-1 analogues at the same mass number.
3) Showing significantly prolonged half-life and stability in pharmacokinetic experiments of the medicaments.
4) High synthetic yield, good stability, easy to scale-up and low cost.

In a specific embodiment, the present invention relates to the following novel agonist polypeptide compounds having the following sequence and side chain modifications result:
Compound 1 (SEQ ID NO. 1): 
His-Aib-Gln-Gly-Thr-Phe-Thr-Ser-Asp-Lys(PEG2-PEG2-γGlu-CO(
CH2)18CO2H)-Ser-Lys-Tyr-Leu-Glu-Aib-Lys-Ala-Ala-Gln-Asp-Phe-Val-
Glu-Trp-Leu-Lys-Ala-Gly-Gly-Pro-Ser-Ser-Gly-Ala-Pro-Pro-Pro-Ser-NH
2
 
H-Aib-QGTFTSDK(PEG2-PEG2-γGlu-CO(CH2)18CO2H)SKYLE-Ai
b-KAAQDFVEWLKAGGPSSGAPPPS-NH2
Compound 2 (SEQ ID NO. 2): 
His-(D-Ser)-Gln-Gly-Thr-Phe-Thr-Ser-Asp-Lys(PEG2-PEG2-CO(C
H2)18CO2H)-Ser-Lys-Tyr-Leu-Glu-Aib-Lys-Ala-Ala-Gln-Asp-Phe-Val-Gl
u-Trp-Leu-Lys-Ala-Gly-Gly-Pro-Ser-Ser-Gly-Ala-Pro-Pro-Pro-Ser-NH2
 
HsQGTFTSDK(PEG2-PEG2-CO(CH2)18CO2H)SKYLE-Aib-KAAQ
DFVEWLKAGGPSSGAPPPS-NH2
Compound 3 (SEQ ID NO. 3): 
His-(D-Ser)-Gln-Gly-Thr-Phe-Thr-Ser-Asp-Lys(PEG2-PEG2-γGlu-C
O(CH2)18CO2H)-Ser-Lys-Tyr-Leu-Glu-Aib-Lys-Ala-Ala-Gln-Asp-Phe-Va
l-Glu-Trp-Leu-Lys-Ala-Gly-Gly-Pro-Ser-Ser-Gly-Ala-Pro-Pro-Pro-Ser-N
H2
 HsQGTFTSDK(PEG2-PEG2-γGlu-CO(CH2)18CO2H)SKYLE-Aib-K AAQDFVEWLKAGGPSSGAPPPS-NH2
Compound 4 (SEQ ID NO. 4): 
His-(D-Ser)-Gln-Gly-Thr-Phe-Thr-Ser-Asp-Lys(PEG2-PEG2-γGlu-C
O(CH2)16CO2H)-Ser-Lys-Tyr-Leu-Glu-Aib-Lys-Ala-Ala-Gln-Asp-Phe-Va
l-Glu-Trp-Leu-Lys-Ala-Gly-Gly-Pro-Ser-Ser-Gly-Ala-Pro-Pro-Pro-Ser-N
H2
 
HsQGTFTSDK(PEG2-PEG2-γGlu-CO(CH2)16CO2H)SKYLE-Aib-K
AAQDFVEWLKAGGPSSGAPPPS-NH2
Compound 5 (SEQ ID NO. 5): 
His-(D-Ser)-Gln-Gly-Thr-Phe-Thr-Ser-Asp-Lys(PEG2-PEG2-γGlu-C
O(CH2)18CH3)-Ser-Lys-Tyr-Leu-Glu-Aib-Lys-Ala-Ala-Gln-Asp-Phe-Val-
Glu-Trp-Leu-Lys-Ala-Gly-Gly-Pro-Ser-Ser-Gly-Ala-Pro-Pro-Pro-Ser-NH
2
 
HsQGTFTSDK(PEG2-PEG2-γGlu-CO(CH2)18CH3)SKYLE-Aib-KA
AQDFVEWLKAGGPSSGAPPPS-NH2
Compound 6 (SEQ ID NO. 6): 
His-(D-Ser)-Gln-Gly-Thr-Phe-Thr-Ser-Asp-Lys(PEG2-PEG2-γGlu-C
O(CH2)18CO2H)-Ser-Lys-Tyr-Leu-Asp-Glu-Arg-Ala-Ala-Gln-Asp-Phe-Iv
a-Gln-Trp-Leu-Leu-Asp-Gly-Gly-Pro-Ser-Ser-Gly-Aib-Pro-Pro-Pro-Ser-
NH2
 
HsQGTFTSDK(PEG2-PEG2-γGlu-CO(CH2)18CO2H)SKYLDERAA
QDF-Iva-QWLLDGGPSSG-Aib-PPPS-NH2
Compound 7 (SEQ ID NO. 7): 
His-(D-Ser)-Gln-Gly-Thr-Phe-Thr-Ser-Asp-Lys(PEG2-PEG2-γGlu-C
O(CH2)18CO2H)-Ser-Lys-Tyr-Leu-Asp-Glu-Arg-Ala-Ala-Gln-Asp-Phe-Iv
a-Gln-Trp-Leu-Leu-Glu-Gly-Gly-Pro-Ser-Ser-Gly-Ala-Pro-Pro-Pro-Ser-
NH2
 
HsQGTFTSDK(PEG2-PEG2-γGlu-CO(CH2)18CO2H)SKYLDERAA
QDF-Iva-QWLLEGGPSSGAPPPS-NH2
Compound 8 (SEQ ID NO. 8): 
His-Aib-Gln-Gly-Thr-Phe-Thr-Ser-Asp-Lys(PEG2-PEG2-γGlu-CO(
CH2)18CO2H)-Ser-Lys-Tyr-Leu-Asp-Glu-Arg-Ala-Ala-Gln-Asp-Phe-Iva-
Gln-Trp-Leu-Leu-Asp-Gly-Gly-Pro-Ser-Ser-Gly-Aib-Pro-Pro-Pro-Ser-N
H2
 
H-Aib-QGTFTSDK(PEG2-PEG2-γGlu-CO(CH2)18CO2H)SKYLDER
AAQDF-Iva-QWLLDGGPSSG-Aib-PPPS-NH2
Compound 9 (SEQ ID NO. 9): 
His-Aib-Gln-Gly-Thr-Phe-Thr-Ser-Asp-Lys(PEG₂-PEG₂-γGlu-CO(
CH₂)₁₆CO₂H)-Ser-Lys-Tyr-Leu-Asp-Glu-Arg-Ala-Ala-Gln-Asp-Phe-Iva-
Gln-Trp-Leu-Leu-Asp-Gly-Gly-Pro-Ser-Ser-Gly-Aib-Pro-Pro-Pro-Ser-N
H₂
 
H-Aib-QGTFTSDK(PEG2-PEG2-γGlu-CO(CH2)16CO2H)SKYLDER
AAQDF-Iva-QWLLDGGPSSG-Aib-PPPS-NH2
Compound 10 (SEQ ID NO. 10): 
His-Aib-Gln-Gly-Thr-Phe-Thr-Ser-Asp-Lys(PEG2-PEG2-γGlu-CO(
CH2)18CH3)-Ser-Lys-Tyr-Leu-Asp-Glu-Arg-Ala-Ala-Gln-Asp-Phe-Iva-Gl
n-Trp-Leu-Leu-Asp-Gly-Gly-Pro-Ser-Ser-Gly-Aib-Pro-Pro-Pro-Ser-NH2
 
H-Aib-QGTFTSDK(PEG2-PEG2-γGlu-CO(CH2)18CH3)SKYLDERA
AQDF-Iva-QWLLDGGPSSG-Aib-PPPS-NH2
Compound 11 (SEQ ID NO. 11): 
His-Aib-Gln-Gly-Thr-Phe-Thr-Ser-Asp-Lys(PEG2-PEG2-CO(CH2)18
CH3)-Ser-Lys-Tyr-Leu-Asp-Glu-Arg-Ala-Ala-Gln-Asp-Phe-Iva-Gln-Trp-
Leu-Leu-Asp-Gly-Gly-Pro-Ser-Ser-Gly-Aib-Pro-Pro-Pro-Ser-NH2
 
H-Aib-QGTFTSDK(PEG2-PEG2-γGlu-CO(CH2)18CH3)SKYLDERA
AQDF-Iva-QWLLDGGPSSG-Aib-PPPS-NH2
Compound 12 (SEQ ID NO. 12): 
His-(D-Ser)-Gln-Gly-Thr-Phe-Thr-Ser-Asp-Lys(PEG2-PEG2-γGlu-C
O(CH2)18CO2H)-Ser-Lys-Tyr-Leu-Asp-Glu-Arg-Ala-Ala-Gln-Asp-Phe-Iv
a-Gln-Trp-Leu-Leu-Asp-Gly-Gly-Pro-Ser-Ser-Gly-Aib-Pro-Pro-Pro-Ser-
NH2
 
HsQGTFTSDK(PEG2-PEG2-γGlu-CO(CH2)18CO2H)SKYLDERAA
QDF-Iva-QWLLDGGPSSG-Aib-PPPS-NH2
Compound 13 (SEQ ID NO. 13): 
His-(D-Ser)-Gln-Gly-Thr-Phe-Thr-Ser-Asp-Lys(Gly-Gly-Ser-Gly-Se
r-Gly-γGlu-CO(CH2)18-COOH)-Ser-Lys-Tyr-Leu-Glu-Aib-Lys-Ala-Ala-
Gln-Asp-Phe-Val-Glu-Trp-Leu-Lys-Ala-Gly-Gly-Pro-Ser-Ser-Gly-Ala-Pr
o-Pro-Pro-Ser
 
HsQGTFTSDK(GGSGSG-γGlu-CO(CH2)18-COOH)SKYLE-Aib-K
AAQDFVEWLKAGGPSSGAPPPS
Compound 14 (SEQ ID NO. 14): 
His-(D-Ser)-Gln-Gly-Thr-Phe-Thr-Ser-Asp-Lys(Gly-Gly-Ser-Gly-Se
r-Gly-γGlu-CO(CH2)18-COOH)-Ser-Lys-Tyr-Leu-Glu-Aib-Lys-Ala-Ala-
Gln-Asp-Phe-Val-Glu-Trp-Leu-Lys-Ala-Gly-Gly-Pro-Ser-Ser-Gly-Ala-Pr
o-Pro-Pro-Ser-NH2
 
HsQGTFTSDK(GGSGSG-γGlu-CO(CH2)18-COOH)SKYLE-Aib-K
AAQDFVEWLKAGGPSSGAPPPS-NH2
Compound 15 (SEQ ID NO. 15): 
His-Aib-Gln-Gly-Thr-Phe-Thr-Ser-Asp-Lys(Gly-Gly-Ser-Gly-Ser-G
ly-γGlu-CO(CH2)18-COOH)-Ser-Lys-Tyr-Leu-Asp-Glu-Arg-Ala-Ala-Gln-
Asp-Phe-Iva-Gln-Trp-Leu-Leu-Asp-Gly-Gly-Pro-Ser-Ser-Gly-Aib-Pro-Pr
o-Pro-Ser
 
H-Aib-QGTFTSDK(GGSGSG-γGlu-CO(CH2)18-COOH)SKYLDER
AAQDF-Iva-QWLLDGGPSSG-Aib-PPPS
Compound 16 (SEQ ID NO. 16):

His-Aib-Gln-Gly-Thr-Phe-Thr-Ser-Asp-Lys(Gly-Gly-Ser-Gly-Ser-G
ly-γGlu-CO(CH2)18-COOH)-Ser-Lys-Tyr-Leu-Asp-Glu-Arg-Ala-Ala-Gln-
Asp-Phe-Iva-Gln-Trp-Leu-Leu-Asp-Gly-Gly-Pro-Ser-Ser-Gly-Aib-Pro-Pr
o-Pro-Ser-NH2

H-Aib-QGTFTSDK(GGSGSG-γGlu-CO(CH2)18-COOH)SKYLDER
AAQDF-Iva-QWLLDGGPSSG-Aib-PPPS-NH2.

In the above sequence, the modification of Lys can be any one of the following structures:

### Lys(PEG₂-PEG₂-γGlu-CO(CH₂)₁₈CO₂H);

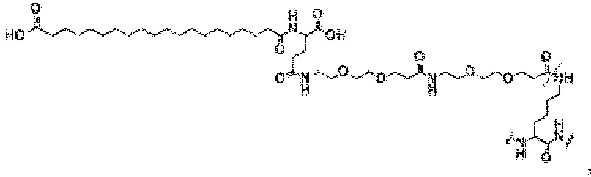

or

### Lys(PEG₂-PEG₂-γGlu-CO(CH₂)₁₈CO₂H);

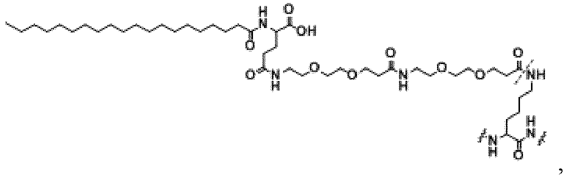

or

### Lys(PEG₂-PEG₂-γGlu-CO(CH₂)₁₂CO₂H);

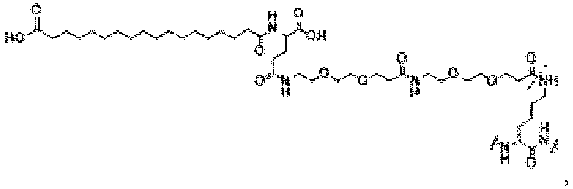

or

### Lys(PEG₂-PEG₂-CO(CH₂)₁₈CO₂H);

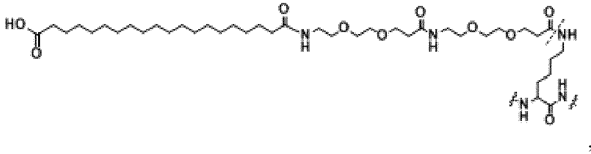

, or

### Lys(Gly-Gly-Ser-Gly-Ser-Gly-γGlu-CO(CH₂)₁₈CO₂H)

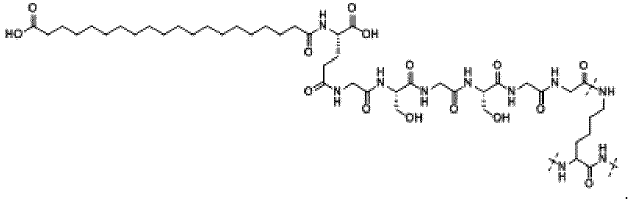

The above Lys attached to a lipophilic substituent can be replaced with:

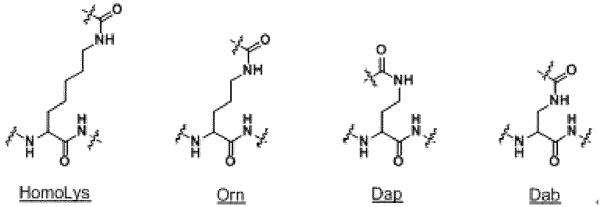

The specific meanings of the abbreviations used in the present invention are as follows.

Boc is tert-butoxycarbonyl, Fmoc is fluorenylmethoxycarbonyl, t-Bu is tert-butyl, ivDDe is 1-(4,4-dimethyl-2,6-dioxo-cyclohexylidene)-3-methyl-butyl, resin is resin, TFA is trifluoroacetic acid, EDT is 1,2-ethanedithiol, Phenol is phenol, FBS is fetal bovine serum, BSA is bovine serum albumin, HPLC is high performance liquid chromatography, GLP-1R is glucagon-like peptide-1 receptor, GCGR is glucagon receptor, GLP-1 is glucagon-like peptide, mPEG is mono-methoxy-polyethylene diol, His is Histidine, Ser is serine, D-Ser is D-type serine, Gln is Glutamine, Gly is Glycine, Glu is Glutamicacud, Ala is Alanine, Thr is Threonine, Lys is Lysine, Arg is Arginine, Tyr is Tyrosine, Asp is Aspartic Acid, Trp is Tryptophan, Phe is Phenylalanine, IIe is Isoleucine, Leu is Leucine, Cys is Cysteine, Pro is Proline, Val is Valine, Met is Methionine, Asn is Asparagine. HomoLys is Homolysine, Orn is ornithine, Dap is diaminoheptanedioic acid, Dab is 2,4-diaminobutyric acid, Aib is 2-aminoisobutyric acid, and Iva is isovaline.

### DESCRIPTION OF THE DRAWINGS

Hereinafter, embodiments of the present invention are described in detail in conjunction with the accompanying drawings, in which:
Fig. 1 shows the area under the curve (AUC) calculated in Example 2, based on the initial blood glucose level of the experimental animal at 4 hours after administration versus the 24-hour traced blood glucose changes at different time points when oral glucose was administered again, p < 0.05.
Fig. 2A shows gel electrophoresis plots of FN expression in different groups detected using Western blot in the in vitro renal fibrosis cell model constructed by high glucose induction of MCS mesangial cells and NRK renal epithelial cells in Example 3.
Fig. 2B shows bar graphs of FN expression in different groups detected by high glucose induction of MCS mesangial cells and NRK renal epithelial cells in Example 3 to construct an in vitro renal fibrosis cell model, p < 0.05; ^{∗}: indicates a confidence level within 95% compared to control (p<0.05); ^{∗∗}: indicatesa confidence level within 99% compared to control (p<0.01).
Fig. 3A shows the blood glucose and body weight of mice measured 8-week-old db/db diabetic mice in Example 4, subcutaneously injected daily or every other day with saline, compound 3 (80 and 120 µg/kg, total 2 groups), compound 8 (80 and 120 µg/kg, total 2 groups), compound 13 (80 and 120 µg/kg, total 2 groups), compound 15 (80 and 120 µg/kg, total 2 groups) and semaglutide (80 and 120µg/kg, total 2 groups) followed by administration and6h fasting on alternate days, p < 0.05.
Fig. 3B shows the blood glucose and body weight of mice measured on 8-week-old db/db diabetic mice in Example 4, subcutaneously injected daily or every other day with saline, compound 3 (80 and 120 µg/kg, 2 groups), compound 8 (80 and 120 µg/kg, 2 groups), compound 13 (80 and 120 µg/kg, 2 groups), compound 15 (80 and 120 µg/kg, 2 groups) and semaglutide (80 and 120µg/kg, total 2 groups) followed by administration and6h fasting on alternate days, p < 0.05..
Fig. 4A shows the weekly blood glucose changes in 12-week-old db/db diabetic mice in Example 4, subcutaneously injected with saline, polypeptide compounds 3, 8, 13, 14, 15, 16 and semaglutide, SAR425899, MED C18-acid, for 6 weeks, respectively.
Fig. 4B shows the OGTT results and corresponding AUC values measured at week 4 in 12-week-old db/db diabetic mice in Example 4, subcutaneously injected with saline, polypeptide compounds 3, 8, 13, 14, 15, 16 and semaglutide, SAR425899, MED C18-acid, respectively.
Fig. 4C shows the ITT results and corresponding AUC values measured at week 5 in 12-week-old db/db diabetic mice of Example 4, subcutaneously injected with saline, polypeptide compounds 3, 8, 13, 14, 15, 16 and semaglutide, SAR425899, MED C18-acid, respectively.
Fig. 4D shows the results of ALT and AST in the serum of 12-week-old db/db diabetic mice in Example 4, subcutaneously injected with saline, polypeptide compounds 3, 8, 13, 14, 15, 16 and semaglutide, SAR425899, MED C18-acid, respectively, p < 0.05.
Fig. 5A shows, from left to right, the urinary protein content, urine glucose content, and serum glycated hemoglobin content, urea nitrogen content, and blood creatinine content in the urine of mice in Example 4 after 8 weeks of treatment.
Fig. 5B shows the H&E staining results of the kidneys of the mice in Example 4 after 8 weeks of medical treatment.
Fig. 5C shows the PAS staining results and statistics of the kidneys of mice in Example 4 after 8 weeks of medical treatment.
Fig. 5D shows the results and the statistical graph of Sirius red staining of the kidneys of mice in Example 4 after 8 weeks of medical treatment.
Fig. 5E shows the results and the statistical graph of FN staining in the kidneys of mice in Example 4after 8 weeks of medical treatment.
Fig. 5F shows the results and statistical graph of α-SMA staining of the kidneys of mice in Example 4 after 8 weeks of medical treatment, p < 0.05.
Fig. 6 shows an exemplary modification of the Lys side chain in the parent peptide of the compound of the present invention.
Fig. 7 shows another exemplary modification of the Lys side chain in the parent peptide of the compound of the present invention.

### DETAILED DESCRIPTION OF THE INVENTION

Embodiments of the invention will be described in detail hereafter in connection with the examples, it will be understood by those skilled in the art that the following examples are only intended to illustrate the invention and should not be considered as limiting the scope of the invention. Where specific conditions are not indicated in the examples, they are carried out according to conventional conditions or those recommended by the manufacturer. Where the manufacturer is not specified for the reagents or apparatus used, they are conventional products available commercially.

### Example 1 Synthesis of polypeptide compounds

Polypeptide compounds 1-16, positive control polypeptide compounds 17-20 (from compounds in Chinese Patent Publication No. CN104926934A), positive control polypeptide compounds 21-26 (from patent CN201711194175.0), SAR425899 and MED C18-acid involved in the present invention were synthesized by the applicant, where SAR425899 and MED C18-acid are both compounds reported in the prior art.

The amino acid sequences of the above positive control polypeptide compounds 17-26, SAR425899 and MED C18-acid compounds are as follows.
Compound 17 (SEQ ID NO. 17): 
His-(D-Ser)-Gln-Gly-Thr-Phe-Thr-Ser-Asp-Tyr-Ser-Lys-Tyr-Leu-As
p-Lys(PEG2-PEG2-γGlu-CO(CH2)14CH3)-Arg-Arg-Ala-Gln-Asp-Phe-Val-
Gln-Trp-Leu-Met-Asn-Thr-Gly-Gly-Pro-Ser-Ser-Gly-Ala-Pro-Pro-Pro-Se
 r-NH2 
HsQGTFTSDYSKYLDK(PEG2-PEG2-γGlu-CO(CH2)14CH3)RRAQ
DFVQWLMNTGGPSSGAPPPS-NH2
Compound 18 (SEQ ID NO. 18): 
His-(D-Ser)-Gln-Gly-Thr-Phe-Thr-Ser-Asp-Tyr-Ser-Lys-Tyr-Leu-As
p-Lys(PEG2-PEG2-γGlu-CO(CH2)14CH3)-Arg-Arg-Ala-Gln-Asp-Phe-Val-
Gln-Trp-Leu-Leu-Asn-Thr-Gly-Gly-Pro-Ser-Ser-Gly-Ala-Pro-Pro-Pro-Se
r-NH2
 
HsQGTFTSDYSKYLDK(PEG2-PEG2-γGlu-CO(CH2)14CH3)RRAQ
DFVQWLLNTGGPSSGAPPPS-NH2
Compound 19 (SEQ ID NO. 19): 
His-(D-Ser)-Gln-Gly-Thr-Phe-Thr-Ser-Asp-Tyr-Ser-Lys(PEG2-PEG2
-CO(CH2)16CO2H)-Lys-Leu-Asp-Aib-Arg-Arg-Ala-Gln-Asp-Phe-Val-Gln
-Trp-Leu-Met-Asn-Thr-Gly-Gly-Pro-Ser-Ser-Gly-Ala-Pro-Pro-Pro-Ser-N
H2
 
HsQGTFTSDYSK(PEG2-PEG2-CO(CH2)16CO2H)KLD-Aib-RRAQ
DFVQWLMNTGGPSSGAPPPS-NH2
Compound 20 (SEQ ID NO. 20): 
His-(D-Ser)-Gln-Gly-Thr-Phe-Thr-Ser-Asp-Tyr-Ser-Lys-Tyr-Leu-As
p-Lys(PEG2-PEG2-γGlu-CO(CH2)14CH3)-Arg-Arg-Ala-Gln-Asp-Phe-Val-
Gln-Trp-Leu-Met-Asn-Thr-Gly-Gly-Pro-Ser-Ser-Gly-Ala-Pro-Pro-Pro-Se
r-NH2
 
H-(d-S)-QGTFTSDYSKYLDK(PEG2-PEG2-γGlu-CO(CH2)14CH3)R
RAQDFVQWLMNTGGPSSGAPPPS-NH2
Compound 21 (SEQ ID NO. 21): 
His-Xaa-Gln-Gly-Thr-Phe-Thr-Ser-Asp-Lys*-Ser-Lys-Tyr-Leu-Asp-
Glu-Gln-Ala-Ala-Gln-Asp-Phe-Val-Gln-Trp-Leu-Leu-Asp-Gly-Pro-Ser-S
er-Gly-Ala-Pro-Pro-Pro-Ser-NH2
 
HXQGTFTSDK*SKYLDEQAAQDFVQWLLDGPSSGAPPPS-NH
2
Compound 22 (SEQ ID NO. 22): 
His-Ser-Gln-Gly-Thr-Phe-Thr-Ser-Asp-Lys*-Ser-Lys-Tyr-Leu-Asp-
Ser-Gln-Ala-Ala-Gln-Asp-Phe-Val-Gln-Trp-Leu-Met-Asn-Gly-Gly-Pro-S
er-Ser-Gly-Ala-Pro-Pro-Pro-Ser-NH2
 
HSQGTFTSDK*SKYLDSQAAQDFVQWLMNGGPSSGAPPPS-N
H2
Compound 23 (SEQ ID NO. 23): 
His-Ser-Gln-Gly-Thr-Phe-Thr-Ser-Asp-Lys*-Ser-Lys-Tyr-Leu-Asp-
Glu-Glu-Ala-Ala-Gln-Asp-Phe-Val-Gln-Trp-Leu-Met-Asn-Gly-Gly-Pro-
Ser-Ser-Gly-Ala-Pro-Pro-Pro-Ser-NH2
 
HSQGTFTSDK*SKYLDEEAAQDFVQWLMNGGPSSGAPPPS-N
H2
Compound 24 (SEQ ID NO. 24): 
His-Ser-Gln-Gly-Thr-Phe-Thr-Ser-Asp-Lys*-Ser-Lys-Tyr-Leu-Asp-
Glu-Gln-Ala-Ala-Gln-Asp-Phe-Val-Gln-Trp-Leu-Met-Asn-Gly-Gly-Pro-
Ser-Ser-Gly-Ala-Pro-Pro-Pro-Ser-NH2
 
HSQGTFTSDK*SKYLDEQAAQDFVQWLMNGGPSSGAPPPS-N
H2
Compound 25 (SEQ ID NO. 25): 
His-Ser-Gln-Gly-Thr-Phe-Thr-Ser-Asp-Lys*-Ser-Lys-Tyr-Leu-Asp-
Glu-Arg-Ala-Ala-Gln-Asp-Phe-Val-Gln-Trp-Leu-Met-Asn-Thr-Gly-Pro-
Ser-Ser-Gly-Ala-Pro-Pro-Pro-Ser-OH
 
HSQGTFTSDK*SKYLDERAAQDFVQWLMNTGPSSGAPPPS-O
H
Compound 26 (SEQ ID NO. 26):

His-Ser-Gln-Gly-Thr-Phe-Thr-Ser-Asp-Lys*-Ser-Lys-Tyr-Leu-Asp-
Ser-Arg-Ala-Ala-Gln-Asp-Phe-Val-Gln-Trp-Leu-Met-Asn-Thr-Gly-Pro-S
er-Ser-Gly-Ala-Pro-Pro-Pro-Ser-OH

HSQGTFTSDK*SKYLDSRAAQDFVQWLMNTGPSSGAPPPS-O
H

Note: X in Compound 21 in the table is aminoisobutyric acid, and K^{∗} in Compound 21-25 indicates that this position is a lysine residue and is covalently linked to a fatty acid whose structure is shown in the following formula: 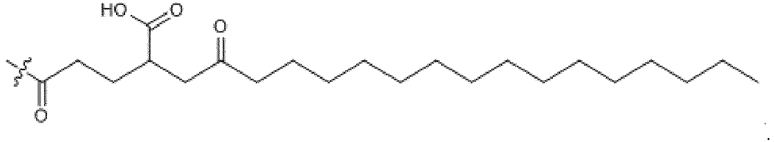
MED C18-acid (SEQ ID NO. 27): 
His-(D-Ser)-Gln-Gly-Thr-Phe-Thr-Ser-Asp-Tyr-Ser-Lys-Lys(PEG2-
PEG2-γGlu-(CH2)17COOH)-Leu-Asp-Ser-Glu-Arg-Ala-Arg-Asp-Phe-Va
l-Ala-Trp-Leu-Val-Ala-Gly-Gly-NH2
 
HsQGTFTSDYSKK(PEG2-PEG2-γGlu-CO(CH2)16CO2H)LDSERA
RDFVAWLVAGG-NH2
SAR425899 (SEQ ID NO. 28):

His-(D-Ser)-Gln-Gly-Thr-Phe-Thr-Ser-Asp-Leu-Ser-Lys-Gln-Lys(C
O(CH2)14CH3)-Glu-Ser-Lys-Ala-Ala-Gln-Asp-Phe-Ile-Glu-Trp-Leu-Lys-
Ala-Gly-Gly-Pro-Ser-Ser-Gly-Ala-Pro-Pro-Pro-Ser-NH2

HsQGTFTSDLSKQK(CO(CH2)14CH3)ESKAAQDFIEWLKAGGPS
SGAPPPS-NH2

To facilitate the demonstration of the preparation method of the above polypeptide compounds, the present invention takes polypeptide compound 3 as an example, and the specific operation steps are as follows.

### (i) Materials:

All amino acids were purchased from NovaBiochem Company. Unless otherwise specified, other reagents were all analytically pure and purchased from Sigma Company. CS336X peptide synthesizer (CS Bio American Company). Phenomenex Luna C18 preparative column (46mm x 250mm) was used to purify the peptides. The high performance liquid chromatograph was manufactured by Waters Company. Mass analysis was determined using Agilent mass spectrometer.

### (ii) Methods:

### 1. Synthesis of polypeptide compound 3.

### Structural sequence:

His-(D-Ser)-Gln-Gly-Thr-Phe-Thr-Ser-Asp-Lys(PEG2-PEG2-γGlu-C
O(CH2)18CO2H)-Ser-Lys-Tyr-Leu-Glu-Aib-Lys-Ala-Ala-Gln-Asp-Phe-Va
l-Glu-Trp-Leu-Lys-Ala-Gly-Gly-Pro-Ser-Ser-Gly-Ala-Pro-Pro-Pro-Ser-N
H2

### 1a) Main peptide chain assembly:

The following peptides were synthesized on a 0.25 mol scale on a CS336X peptide synthesizer (CS Bio American Company) according to the Fmoc/t-Bu strategy:
Boc-His(Boc)-D-Ser(t-Bu)-Gln(Trt)-Gly-Thr(t-Bu)-Phe-Thr(t-Bu)-S er(tBu)-Asp(OtBu)-Lys(PEG₂-PEG₂-γGlu-CO(CH₂)₁₈CO₂H)-Ser(t-Bu)-L ys(Boc)-Tyr(t-Bu)-Leu-Glu(OtBu)-Aib-Lys(Boc)-Ala-Ala-Gln(Trt)-Asp( OtBu)-Phe-Val-Glu(OtBu)-Trp(Boc)-Leu-Lys(Boc)-Ala-Gly-Gly-Pro-Ser (t-Bu)-Ser(t-Bu)-Gly-Ala-Pro-Pro-Ser(t-Bu)-rink amide resin
(1) Step 1: 0.75 g of Rink amide resin was dissolved in dichloromethane and the resin was washed with N,N-dimethylformamide for three times.
(2) Step 2: The procedure reaction was performed using Rink amide resin as carrier, the mixture of benzotriazole-N,N,N',N'-tetramethyluronium hexafluorophosphate, 1-hydroxybenzotriazole and N,N-diisopropylethylamine in the ratio of the amount of substance (molar ratio) of 1: 1: 1 was used as the coupling agent, and N,N-dimethylformamide was used as the solvent, the procedure reaction was carried out and the condensation reaction was performed sequentially to couple Fmoc-Ser(t-Bu)-OH, Fmoc-Pro-OH (3x), Fmoc-Ala-OH, Fmoc-Gly-OH, Fmoc-Ser(t-Bu)-OH (2x), Fmoc-Pro-OH, Fmoc-Gly-OH (2x), Fmoc-Ala-OH, Fmoc-Lys (Boc)-OH, Fmoc-Leu-OH, Fmoc-Trp(Boc)-OH, Fmoc-Glu(OtBu)-OH, Fmoc-Val-OH, Fmoc-Phe-OH, Fmoc-Asp(OtBu)-OH, Fmoc-Gln(Trt)-OH, Fmoc-Ala-OH (2x) , Fmoc-Lys(Boc)-OH, Fmoc-Aib-OH, Fmoc-Glu(OtBu)-OH, Fmoc-Leu-OH, Fmoc-Tyr(t-Bu)-OH, Fmoc-Lys(Boc)-OH, Fmoc-Ser(t-Bu)-OH, Fmoc-Lys(PEG₂-PEG₂-γGlu-CO(CH₂)₁₈CO₂H)-OH, Fmoc-Asp(OtBu)-OH, Fmoc-Ser(t-Bu)-OH, Fmoc-Thr(t-Bu)-OH, Fmoc-Phe-OH, Fmoc-Thr(t-Bu)-OH, Fmoc-Gly-OH, Fmoc- Gln(Trt)-OH, Fmoc-D-Ser(t-Bu)-OH, Boc-His(Boc)-OH to obtain:
   Boc-His(Boc)-D-Ser(t-Bu)-Gln(Trt)-Gly-Thr(t-Bu)-Phe-Thr(t-Bu)-S er(tBu)-Asp(OtBu)-Lys(PEG₂-PEG₂-γGlu-CO(CH₂)₁₈CO₂H)-Ser(t-Bu)-L ys(Boc)-Tyr(t-Bu)-Leu-Glu(OtBu)-Aib-Lys(Boc)-Ala-Ala-Gln(Trt)-Asp( OtBu)-Phe-Val-Glu(OtBu)-Trp(Boc)-Leu-Lys(Boc)-Ala-Gly-Gly-Pro-Ser (t-Bu)-Ser(t-Bu)-Gly-Ala-Pro-Pro-Pro-Ser(t-Bu)-rink amide resin, wherein the ratio of the amount of substance (molar ratio) of the amount of the Fmoc-protected amino acids fed to the amount of resin used in each condensation reaction was 1:1 to 6:1, and the amount of Fmoc-protected amino acids fed to the amount of resin used in each condensation reaction was determined by the ratio of the amount of substance (molar ratio) of the amount of benzotriazole-N,N,N',N'-tetramethylformamidiniumhexafluorophosphatef ed to the amount of Fmoc-protected amino acids used in each condensation reaction was 3:1.

### 1b) Removal of Lys(PEG₂-PEG₂-γGlu-CO(CH₂)₁₈CO₂H) and introduction of lipophilic substituents:

The protected peptide-based resin synthesized in 1a) was washed twice in a mixture of N-methylpyrrolidone:/dichloromethane (DCM) = 1:1 (v/v), freshly prepared 2.0% solution of hydrazine hydrate N-methylpyrrolidone was added, and the reaction mixture was shaken for 12.0 min at room temperature and then filtered. The hydrazine treatment step was repeated twice to obtain:
Boc-His(Boc)-D-Ser(t-Bu)-Gln(Trt)-Gly-Thr(t-Bu)-Phe-Thr(t-Bu)-S er(tBu)-Asp(OtBu)-Lys-Ser(t-Bu)-Lys(Boc)-Tyr(t-Bu)-Leu-Glu(OtBu)-Ai b-Lys(Boc)-Ala-Ala-Gln(Trt)-Asp(OtBu)-Phe-Val-Glu(OtBu)-Trp(Boc)-Leu-Lys(Boc)-Ala-Gly-Gly-Pro-Ser(t-Bu)-Ser(t-Bu)-Gly-Ala-Pro-Pro-Pr o-Ser(t-Bu)-rink amide resin. Thereafter the resin was washed well with dichloromethane and N-methylpyrrolidone. A mixed coupling solution of FmocNH-PEG₂-OH, benzotriazole-N,N,N',N'-tetramethyluronium hexafluorophosphate, 1-hydroxybenzotriazole, N-methylpyrrolidone with diisopropylethylamine was added thereto and shaken for 3.0 h, filtered and washed, the hydrazine treatment step was repeated twice to yield: Boc-His(Boc)-D-Ser(t-Bu)-Gln(Trt)-Gly-Thr(t-Bu)-Phe-Thr(t-Bu)-Ser(tB u)-Asp(OtBu)-Lys(PEG₂-OH)-Ser(t-Bu)-Lys(Boc)-Tyr(t-Bu)-Leu-Glu(Ot Bu)-Aib-Lys(Boc)-Ala-Ala-Gln(Trt)-Asp(OtBu)-Phe-Val-Glu(OtBu)-Trp( Boc)-Leu-Lys(Boc)-Ala-Gly-Gly-Pro-Ser(t-Bu)-Ser(t-Bu)-Gly-Ala-Pro-P ro-Pro-Ser(t-Bu)-rink amide resin. The Fmoc group was removed from the piperidine/N,N-dimethylformamide solution and the Fmoc-PEG₂-OH coupling reaction was repeated once to yield: Boc-His(Boc)-D-Ser(t-Bu)-Gln(Trt)-Gly-Thr(t-Bu)-Phe-Thr(t-Bu)-Ser(tB u)-Asp(OtBu)-Lys(PEG₂-PEG₂-OH)-Ser(t-Bu)-Lys(Boc)-Tyr(t-Bu)-Leu-Glu(OtBu)-Aib-Lys(Boc)-Ala-Ala-Gln(Trt)-Asp(OtBu)-Phe-Val-Glu(OtB u)-Trp(Boc)-Leu-Lys(Boc)-Ala-Gly-Gly-Pro-Ser(t-Bu)-Ser(t-Bu)-Gly-Al a-Pro-Pro-Pro-Ser(t-Bu)-rink amide resin. The Fmoc group was removed from piperidine/N,N-dimethylformamide solution, and then the Fmoc-yGlu-OtBu, tBu monoprotected eicosanoic fatty acids were sequentially coupled according to conventional conditions to yield: Boc-His(Boc)-D-Ser(t-Bu)-Gln(Trt)-Gly-Thr(t-Bu)-Phe-Thr(t-Bu)-Ser(tB u)-Asp(OtBu)-Lys(PEG₂-PEG₂-γGlu-CO(CH₂)₁₈CO₂H)-Ser(t-Bu)-Lys(Bo c)-Tyr(t-Bu)-Leu-Glu(OtBu)-Aib-Lys(Boc)-Ala-Ala-Gln(Trt)-Asp(OtBu) -Phe-Val-Glu(OtBu)-Trp(Boc)-Leu-Lys(Boc)-Ala-Gly-Gly-Pro-Ser(t-Bu) -Ser(t-Bu)-Gly-Ala-Pro-Pro-Pro-Ser(t-Bu)-rink amide resin.

1c) Removal of total peptide protection:
Boc-His(Boc)-D-Ser(t-Bu)-Gln(Trt)-Gly-Thr(t-Bu)-Phe-Thr(t-Bu)-S er(tBu)-Asp(OtBu)-Lys(PEG₂-PEG₂-γGlu-CO(CH₂)₁₈CO₂H)-Ser(t-Bu)-L ys(Boc)-Tyr(t-Bu)-Leu-Glu(OtBu)-Aib-Lys(Boc)-Ala-Ala-Gln(Trt)-Asp( OtBu)-Phe-Val-Glu(OtBu)-Trp(Boc)-Leu-Lys(Boc)-Ala-Gly-Gly-Pro-Ser (t-Bu)-Ser(t-Bu)-Gly-Ala-Pro-Pro-Pro-Ser(t-Bu)-rink amide resin was added to a round bottom flask, and the reaction was carried out under ice bath conditions by adding cutting solution TFA/EDT/Phenol/H₂O (88/2/5/5, v/v) to raise the temperature and the temperature of the lysate was controlled at 25°C for 120 min. Filtered, the filter cake was washed 3 times with a small amount of trifluoroacetic acid and the filtrate was combined. The filtrate was slowly poured into ice ether under stirring, and was allowed to stand for more than 1.0 hour till complete precipitation. The supernatant was decanted off and the precipitate was centrifuged and washed 6 times with ice ether to obtain the crude compound:

His-(D-Ser)-Gln-Gly-Thr-Phe-Thr-Ser-Asp-Lys(PEG2-PEG2-γGlu-CO(C
H2)18CO2H)-Ser-Lys-Tyr-Leu-Glu-Aib-Lys-Ala-Ala-Gln-Asp-Phe-Val-Gl
u-Trp-Leu-Lys-Ala-Gly-Gly-Pro-Ser-Ser-Gly-Ala-Pro-Pro-Pro-Ser-NH2

Id) Refinement and purification of polypeptide compounds:
The crude product obtained in 1c was dissolved in 5.0% acetic acid in a solution of acetonitrile:H₂O = 1:1 (v/v) and purified by semi-preparative HPLC on a reversed-phase C18 packed 50 mm x 250 mm column for 2 times. The column was eluted with 30-60% acetonitrile-0.1% trifluoroacetic acid/H₂O gradient at 40 mL/min for 45.0 min. The fraction containing peptides was collected, concentrated to remove acetonitrile and then lyophilized. The pure product with >95% purity by HPLC was obtained. The separated product was analyzed by liquid-mass spectrometry, and the m/z value of the ion peak of the protonated molecule was found to be: 4860.9, while the theoretical value was 4863.5.

**Table 1. Polypeptide compounds of the present invention**

| **Compou nd** | **Parent peptide** | **Structure** | **Theore tical value** | **Measu red value** |
|---|---|---|---|---|
| 1 | SEQ ID NO. 1 | His-Aib-Gln-Gly-Thr-Phe-Thr-Ser-Asp- Lys(PEG2-PEG2-γGlu-CO(CH2)18CO2H)- Ser-Lys-Tyr-Leu-Glu-Aib-Lys-Ala-Ala- Gln-Asp-Phe-Val-Glu-Trp-Leu-Lys-Ala- Gly-Gly-Pro-Ser-Ser-Gly-Ala-Pro-Pro-Pr o-Ser-NH2 | 4861.5 | 4858.5 |
| 2 | SEQ ID NO. 2 | His-(D-Ser)-Gln-Gly-Thr-Phe-Thr-Ser-A sp-Lys(PEG2-PEG2-CO(CH2)18CO2H)-Se r-Lys-Tyr-Leu-Glu-Aib-Lys-Ala-Ala-Gln -Asp-Phe-Val-Glu-Trp-Leu-Lys-Ala-Gly- Gly-Pro-Ser-Ser-Gly-Ala-Pro-Pro-Pro-Se r-NH2 | 4734.4 | 4731.5 |
| 3 | SEQ ID NO. 3 | His-(D-Ser)-Gln-Gly-Thr-Phe-Thr-Ser-A sp-Lys(PEG2-PEG2-γGlu-CO(CH2)18CO2 H)-Ser-Lys-Tyr-Leu-Glu-Aib-Lys-Ala-Al a-Gln-Asp-Phe-Val-Glu-Trp-Leu-Lys-Al a-Gly-Gly-Pro-Ser-Ser-Gly-Ala-Pro-Pro- Pro-Ser-NH2 | 4863.5 | 4860.9 |
| 4 | SEQ ID NO. 4 | His-(D-Ser)-Gln-Gly-Thr-Phe-Thr-Ser-A sp-Lys(PEG2-PEG2-γGlu-CO(CH2)16CO2 H)-Ser-Lys-Tyr-Leu-Glu-Aib-Lys-Ala-Al a-Gln-Asp-Phe-Val-Glu-Trp-Leu-Lys-Al a-Gly-Gly-Pro-Ser-Ser-Gly-Ala-Pro-Pro- Pro-Ser-NH2 | 4835.5 | 4832.5 |
| 5 | SEQ ID NO. 5 | His-(D-Ser)-Gln-Gly-Thr-Phe-Thr-Ser-A sp-Lys(PEG₂-PEG₂-γGlu-CO(CH₂)₁₈CH₃ )-Ser-Lys-Tyr-Leu-Glu-Aib-Lys-Ala-Ala- Gln-Asp-Phe-Val-Glu-Trp-Leu-Lys-Ala- Gly-Gly-Pro-Ser-Ser-Gly-Ala-Pro-Pro-Pr o-Ser-NH₂ | 4833.5 | 4830.5 |
| 6 | SEQ ID NO. 6 | His-(D-Ser)-Gln-Gly-Thr-Phe-Thr-Ser-A sp-Lys(PEG2-PEG2-γGlu-CO(CH2)18CO2 H)-Ser-Lys-Tyr-Leu-Asp-Glu-Arg-Ala-A la-Gln-Asp-Phe-Iva-Gln-Trp-Leu-Leu-A sp-Gly-Gly-Pro-Ser-Ser-Gly-Aib-Pro-Pr o-Pro-Ser-NH2 | 4963.5 | 4960.5 |
| 7 | SEQ ID NO. 7 | His-(D-Ser)-Gln-Gly-Thr-Phe-Thr-Ser-A sp-Lys(PEG2-PEG2-γGlu-CO(CH2)18CO2 H)-Ser-Lys-Tyr-Leu-Asp-Glu-Arg-Ala-A la-Gln-Asp-Phe-Iva-Gln-Trp-Leu-Leu-Gl u-Gly-Gly-Pro-Ser-Ser-Gly-Ala-Pro-Pro- Pro-Ser-NH2 | 4963.5 | 4960.9 |
| 8 | SEQ ID NO. 8 | His-Aib-Gln-Gly-Thr-Phe-Thr-Ser-Asp- Lys(PEG2-PEG2-γGlu-CO(CH2)18CO2H)- Ser-Lys-Tyr-Leu-Asp-Glu-Arg-Ala-Ala- Gln-Asp-Phe-Iva-Gln-Trp-Leu-Leu-Asp- Gly-Gly-Pro-Ser-Ser-Gly-Aib-Pro-Pro-P ro-Ser-NH2 | 4961.6 | 4959.3 |
| 9 | SEQ ID NO. 9 | His-Aib-Gln-Gly-Thr-Phe-Thr-Ser-Asp- Lys(PEG2-PEG2-γGlu-CO(CH2)16CO2H)- Ser-Lys-Tyr-Leu-Asp-Glu-Arg-Ala-Ala- Gln-Asp-Phe-Iva-Gln-Trp-Leu-Leu-Asp- Gly-Gly-Pro-Ser-Ser-Gly-Aib-Pro-Pro-P ro-Ser-NH2 | 4933.5 | 4930.4 |
| 10 | SEQ ID NO. 10 | His-Aib-Gln-Gly-Thr-Phe-Thr-Ser-Asp- | 4931.6 | 4928.6 |
| | | Lys(PEG2-PEG2-γGlu-CO(CH2)18CH3)-S er-Lys-Tyr-Leu-Asp-Glu-Arg-Ala-Ala-Gl n-Asp-Phe-Iva-Gln-Trp-Leu-Leu-Asp-Gl y-Gly-Pro-Ser-Ser-Gly-Aib-Pro-Pro-Pro- Ser-NH2 | | |
| 11 | SEQ ID NO. 11 | His-Aib-Gln-Gly-Thr-Phe-Thr-Ser-Asp- Lys(PEG2-PEG2-CO(CH2)18CH3)-Ser-Ly s-Tyr-Leu-Asp-Glu-Arg-Ala-Ala-Gln-As p-Phe-Iva-Gln-Trp-Leu-Leu-Asp-Gly-Gl y-Pro-Ser-Ser-Gly-Aib-Pro-Pro-Pro-Ser- NH2 | 4802.5 | 4799.5 |
| 12 | SEQ ID NO. 12 | His-(D-Ser)-Gln-Gly-Thr-Phe-Thr-Ser-A sp-Lys(PEG2-PEG2-γGlu-CO(CH2)18CO2 H)-Ser-Lys-Tyr-Leu-Asp-Glu-Arg-Ala-A la-Gln-Asp-Phe-Iva-Gln-Trp-Leu-Leu-A sp-Gly-Gly-Pro-Ser-Ser-Gly-Aib-Pro-Pr o-Pro-Ser-NH2 | 4963.5 | 4960.6 |
| 13 | SEQ ID NO. 13 | His-(D-Ser)-Gln-Gly-Thr-Phe-Thr-Ser-A sp-Lys(Gly-Gly-Ser-Gly-Ser-Gly-γGlu-C O(CH2)18-COOH)-Ser-Lys-Tyr-Leu-Glu- Aib-Lys-Ala-Ala-Gln-Asp-Phe-Val-Glu- Trp-Leu-Lys-Ala-Gly-Gly-Pro-Ser-Ser-G ly-Ala-Pro-Pro-Pro-Ser | 4948.5 | 4948.8 |
| 14 | SEQ ID NO. 14 | His-(D-Ser)-Gln-Gly-Thr-Phe-Thr-Ser-A sp-Lys(Gly-Gly-Ser-Gly-Ser-Gly-γGlu-C O(CH2)18-COOH)-Ser-Lys-Tyr-Leu-Glu- Aib-Lys-Ala-Ala-Gln-Asp-Phe-Val-Glu- Trp-Leu-Lys-Ala-Gly-Gly-Pro-Ser-Ser-G ly-Ala-Pro-Pro-Pro-Ser-NH2 | 4963.5 | 4962.6 |
| 15 | SEQ ID NO. 15 | His-Aib-Gln-Gly-Thr-Phe-Thr-Ser-Asp- Lys(Gly-Gly-Ser-Gly-Ser-Gly-γGlu-CO( | 5046.4 | 5046. 8 |
| | | CH2)18-COOH)-Ser-Lys-Tyr-Leu-Asp-Gl u-Arg-Ala-Ala-Gln-Asp-Phe-Iva-Gln-Tr p-Leu-Leu-Asp-Gly-Gly-Pro-Ser-Ser-Gl y-Aib-Pro-Pro-Pro-Ser | | |
| 16 | SEQ ID NO. 16 | His-Aib-Gln-Gly-Thr-Phe-Thr-Ser-Asp- Lys(Gly-Gly-Ser-Gly-Ser-Gly-γGlu-CO( CH2)18-COOH)-Ser-Lys-Tyr-Leu-Asp-Gl u-Arg-Ala-Ala-Gln-Asp-Phe-Iva-Gln-Tr p-Leu-Leu-Asp-Gly-Gly-Pro-Ser-Ser-Gl y-Aib-Pro-Pro-Pro-Ser-NH2 | 5061.4 | 5060.6 |

### Example 2 Effects of polypeptide compounds 1-16, positive control polypeptide compounds 17-26, liraglutide and semaglutide on oral glucose tolerance (OGTT)

Semaglutide was purchased from Zhejiang Paitai Biological Co., Ltd (CAS No.: 910463-68-2) and Liraglutide was purchased from Shenzhen Jianyuan Pharmaceutical Technology Co., Ltd. (CAS No.: 204656-20-2).

Eight-week-old male C57BL/6J mice (Model Animal Research Center, Nanjing University) were randomly grouped according to similar blood glucose (assessed by blood samples obtained from the tail tip), with six mice in each group.

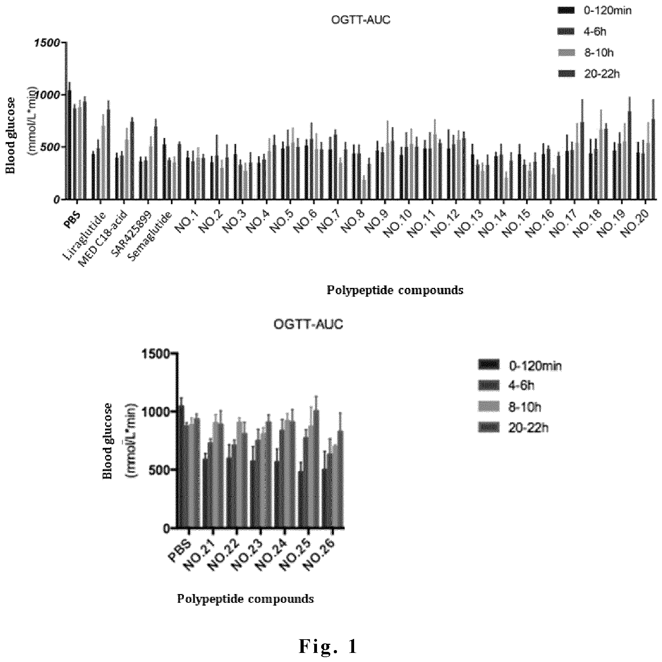

After fasting (6 h), the animals were administered with polypeptide compounds 1-16, positive control polypeptide compounds 17-26, liraglutide, semaglutide, SAR425899 and MED C18-acid in the present invention at a dose of 100µg/kg, subcutaneously, while the control group was administered with PBS. The initial blood samples (fasted blood glucose levels) were obtained after about 4 h. Subsequently, an oral dose of glucose (2 g/kg) was administered and the animals were placed back in their holding cages (t=0). Blood glucose was measured at t=15 min, t=30 min, t=60 min and t=120 min. The oral dose of glucose was then repeated at 8h of administration, 12h of administration and 24h of administration, and blood glucose changes were detected and followed until 24 hours. The data were processed using the software GraphPadPrism to make a line graph of blood glucose changes, and the area under the curve was calculated to obtain an AUC graph, and the results are shown in Fig.1.

Compared to the carrier agent (control PBS), liraglutide and compounds 17-26 could have a significantly lower AUC (p<0.05) in the first OGTT curve period (0-120 min), while in the next three OGTT curve periods (4-22 h), their AUC started to elevate. In contrast, the AUC of semaglutide was significantly reduced (P<0.05) in all four OGTT curves, indicating its long-lasting hypoglycemic effect. And compared with the carrier agent (control PBS), the polypeptide compounds of the present invention all showed different degrees of improvement in glucose tolerance in mice, with polypeptide compounds 1-16 exhibiting a more excellent and significant improvement in glucose tolerance at four OGTT curve periods (0-22h), while the results also indicate that polypeptide compounds 1-16, compared with polypeptide compounds 17-26, liraglutide SAR425899 and MED C18-acid have more excellent and significant long-lasting hypoglycemic effects. Among them, polypeptide compounds 1, 2, 3, 8, 13, 14 and 15 exhibited superior and significant long-acting hypoglycemic effects compared to semaglutide at four OGTT curves (0-22h).

### Example 3 Amelioration of renal fibrosis by polypeptide compounds 1, 2, 3, 8, 13, 14, and 15 in a high glucose-induced renal fibrosis model

Hyperglycemia and increased production of glycosylation end products will cause morphological changes such as proliferation and hypertrophy of glomerular mesangial cells, increase in extracellular matrix (ECM) and mesangial expansion, which will eventually lead to the loss of its physiological function. In addition, trans-differentiation of renal tubular epithelial cell is also an important pathological basis of renal fibrosis, and it has been found that high concentration of glucose further induces glomerular fibrosis by activating the RAS system. Therefore, this example used high glucose to induce MCS mesangial cells and NRK renal epithelial cells to construct an in vitro renal fibrosis cell model for screening medicaments useful for the treatment of diabetic nephropathy.

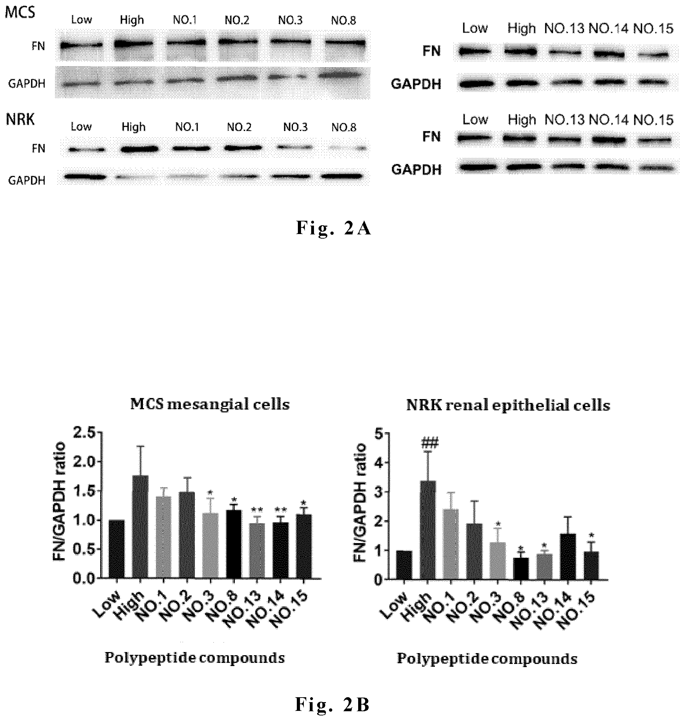

The renal mesangial cell strain GMC and glomerular epithelial cells mTEC used in this example were both cells kept in the applicant's laboratory (both were extracted from rat renal primary cells) and cultured in low sugar medium containing 10% fetal bovine serum. The cells were starved with serum-free culture medium for 24 h when grew to 70% confluence. The cells were divided into the following 9 groups by adjusting the glucose concentration as follows: (i) Low group (5.5 mmol/L glucose), (ii) High group (30 mmol/L glucose), (iii) High glucose + compound 1 group (30 mmol/L glucose + NO. 1 10µM), (iv) High glucose + compound 2 group(30 mmol/L glucose + NO. 2 10µM), (v) high sugar + compound 3 group (30mmol/L glucose + NO.3 10µM), (vi) high sugar + compound 8 group (30mmol/L glucose + NO.8 10µM), (vii)high sugar + compound 13 group (30mmol/L glucose + NO.13 10µM), (viii) high sugar + compound 14 group (30mmol/L glucose + NO.14 10µM), and (ix) high sugar + compound 15 group (30mmol/L glucose + NO.15 10µM) were incubated at 37°C 5% CO₂ for 48h for Western blot assay of FN protein (fibronectin). As shown in Fig. 2A, the gel electrophoresis graphs of FN expression in different groups detected by Western blot was shown in an in vitro renal fibroblast cell model constructed by high glucose induction of MCS mesangial cells and NRK renal epithelial cells in this example; as shown in Fig. 2B, the bar graphs of FN expression detected in different groups was shown in the in vitro renal fibroblast cell model constructed by high glucose induction of MCS mesangial cells and NRK renal epithelial cells in this example 3.

According to the previous experimental study and previous literature, 30 mmol/L glucose can induce the hypertrophy of glomerular mesangial cells and the trans-differentiation of renal epithelial cells. Western blot assay results showed that the expression level of FN protein was higher in the High group than the control Low group under high glucose stimulation; while all the administered groups can down-regulate the expression of FN protein, suggesting that the polypeptide compound of the present invention can significantly reduce the increase of ECM caused by high glucose. Among them, polypeptide compounds 3, 8, 13, 14 and 15 inhibited FN expression more significantly.

### Example 4 Therapeutic effects of polypeptide compounds 3, 8, 13, 14, 15 and 16, semaglutide, SAR425899, MED C18-acid on diabetic mice

### (i) Experiments for the determination of different concentrations of medicaments of polypeptide compounds 3, 8, 13 and 15

The db/db mice diabetic model (purchased from Nanjing University-Nanjing Institute of Biomedical Research, about 8 weeks, and blood glucose and body weight were measured to ensure the smooth subsequent experiments) was obtained, and the model mice were randomly divided into 12 groups (including polypeptide compounds 3, 8, 13, 15, semaglutide and physiological saline groups), with 6 mice in each group and no difference in basal body weight and blood glucose. Each group was injected subcutaneously with compound 3 (80 and 120µg/kg, 2 groups), compound 8 (80 and 120µg/kg, 2 groups), compound 13 (80 and 120µg/kg, 2 groups), compound 15 (80 and 120µg/kg, 2 groups), semaglutide (80 and 120µg/kg, 2 groups) and physiological saline (DN group: normal mice born in the same litter; DC group: db/db mice control group) every day or every other day.

Blood glucose and body weight of mice were measured after daily administration and 6h fasting on alternate days.

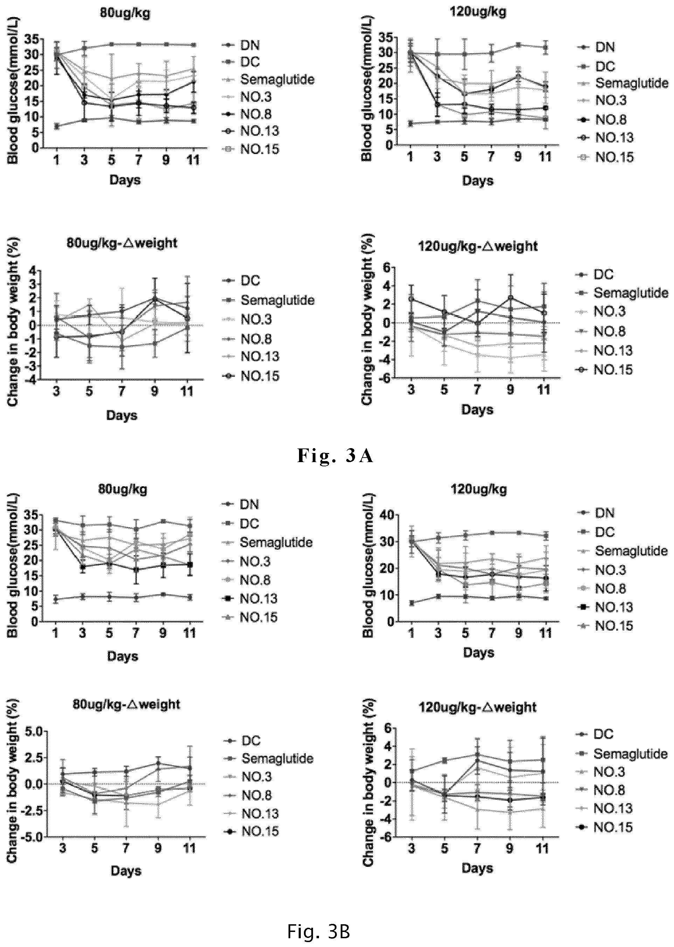

Fig. 3A shows that compared with the physiological saline group DC group, the semaglutide group (120µg/kg) exhibited a better hypoglycemic effect, after which it was maintained at a more stable normoglycemic level, but its hypoglycemic effect was not significant. In the administration group, polypeptide compounds 3, 8, 13 and 15 at a dose of 80µg/kg, polypeptide compounds 3 and 8 showed significant glucose-lowering effect, and polypeptide compounds 13 and 15 showed significant glucose-lowering effect with long-lasting effect; at a dose of 120µg/kg, polypeptide compounds 3, 8, 13 and 15 already achieved good glucose-lowering effect in 3-5 days, and their blood glucose was stably maintained at normal level, besides, polypeptide compounds 3, 8, 13 and 15 showed better weight-lowering effect, and the effect of compounds 13 and 15 was superior to that of compounds 3 and 8. Therefore, the medicament concentration was set at 120µg/kg.

Blood glucose and body weight of mice were measured after daily administration and 6h fasting on alternate days.

Fig. 3B shows that compared with the physiological saline group DC group, the semaglutide group had a hypoglycemic effect at the dose of 80µg/kg and 120µg/kg every other day, but the hypoglycemic effect was not significant, and its hypoglycemic effect was not significant. In the administration group of polypeptide compounds 3 and 8, at the dose of 80µg/kg, the blood glucose of polypeptide compounds 3 and 8 showed a first decrease and then increase, and the overall hypoglycemic effect was significant, and polypeptide compounds 3 and 8 had a hypoglycemic effect. While polypeptide compounds 13 and 15 had significant hypoglycemic effect on day 3, but the subsequent blood glucose level did not change much and tended to be stable; at the dose of 120µg/kg, the blood glucose of semaglutide group rebounded on day 3, and its long-lasting effect was not enough. Polypeptide compounds 3, 8, 13 and 15 had achieved good hypoglycemic effect on days 3-5, and after then, the blood glucose did not change much for polypeptide compounds 3 and 8. Polypeptide compounds 13 and 15 kept good hypoglycemic effect, which proved that polypeptide compounds 13 and 15 have good hypoglycemic long-lasting effect. Meanwhile, polypeptide compounds 3, 8, 13 and 15 showed more excellent hypoglycemic effect, and the effect of polypeptide compounds 13 and 15 was better than that of polypeptide compounds 3 and 8. Therefore, the medicament concentration was set at 120µg/kg.

### (II) Ameliorating effects of polypeptide compounds 3, 8, 13, 14, 15, 16, semaglutide, SAR425899 and MED C18-acid on diabetes in diabetic mice

A db/db mouse model of diabetes (purchased from Nanjing University-Nanjing Institute of Biomedical Research, mice at about 12 weeks, and blood glucose and body weight were measured to ensure that the subsequent experiments were carried out smoothly) was obtained, and the model mice were randomly divided into 10 groups (polypeptide compounds 3, 8, 13, 14, 15, 16, semaglutide, SAR425899, MED C18-acid and saline group), with 6 mice in each group, and no difference in basal body weight and blood glucose in each mouse, each group was injected subcutaneously daily with polypeptide compounds 3, 8, 13, 14, 15, and 16 (120 µg/kg), semaglutide (120 µg/kg), SAR425899 (120 µg/kg), MED C18-acid (120 µg/kg), and saline (DN group. Normal mice born in the same litter; DC group: db/db mice control group). Blood glucose and body weight were measured on alternate days after daily administration of the medicament, and water intake and food intake were measured once a week. OGTT was measured at week 4, insulin tolerance (ITT) was measured at week 5, metabolism of mice was examined using mouse metabolic cages at weeks 6-7, and various serological and pathological indices were taken at week 8.

### 1. The effect of the medicaments on the glucose lowering weight and improvement of insulin resistance in diabetic mice

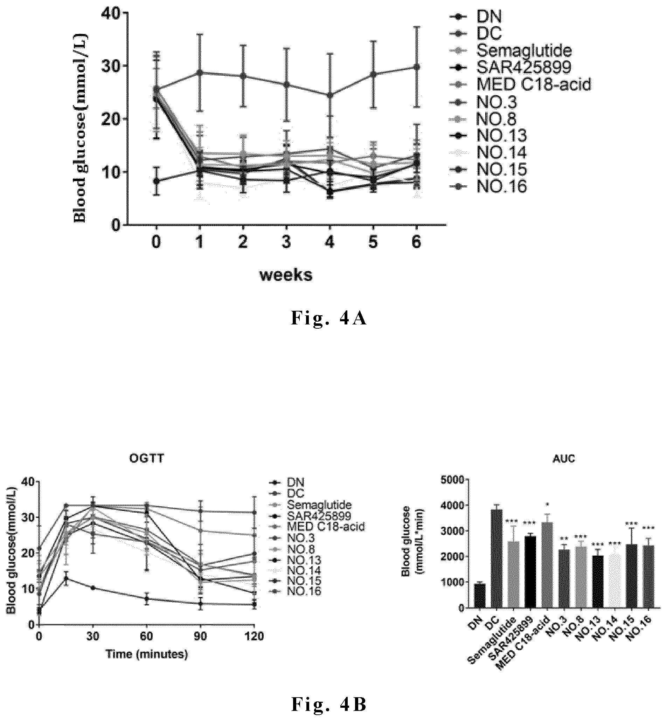

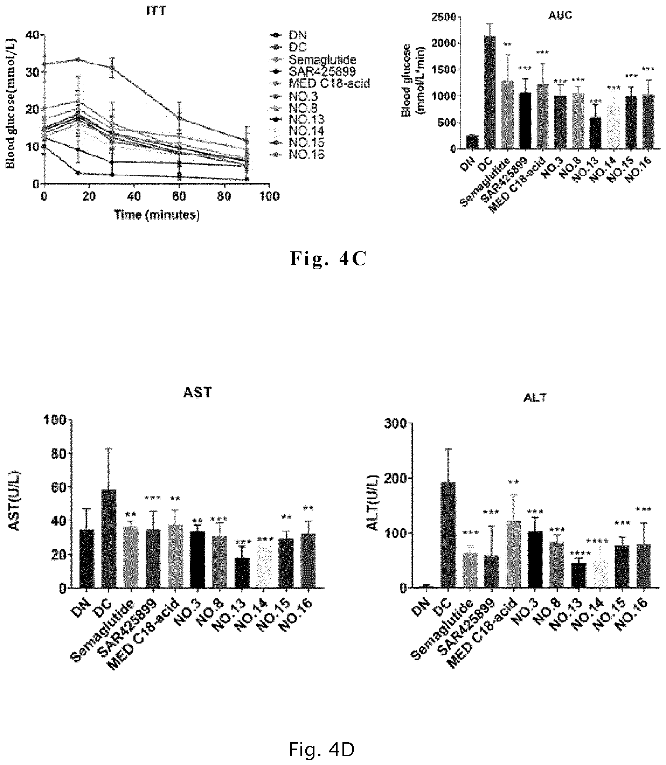

Type 2- diabetes mice were characterized by obesity, insulin resistance, hyperglycemia, dyslipidemia and hepatic fat vacuole-like degeneration. Oral glucose tolerance test (OGTT) and insulin tolerance test (ITT) were performed in diabetic mice treated with polypeptide compounds 3, 8, 13, 14, 15, 16 and semaglutide, SAR425899, MED C18-acid at 4 and 5 weeks, respectively, and the results are shown in Figs. 4A-4D.

The results in Figs. 4A to 4C showed that polypeptide compounds 3, 8, 13, 14, 15, 16, semaglutide, SAR425899 and MED C18-acid all had significant hypoglycemic effects compared to the DN group, with blood glucose already falling to normal levels at week 1 and relatively stable thereafter. However, the polypeptide compounds 3, 8, 13, 14, 15 and 16 performed more excellent in improving oral glucose tolerance (OGTT) and insulin resistance (ITT) in diabetic mice.

In addition, the results in Fig. 4D showed that polypeptide compounds 3, 8, 13, 14, 15, and 16 had more excellent hepatoprotective functions.

### 2. Therapeutic effect of the medicaments on diabetic nephropathy in diabetic mice

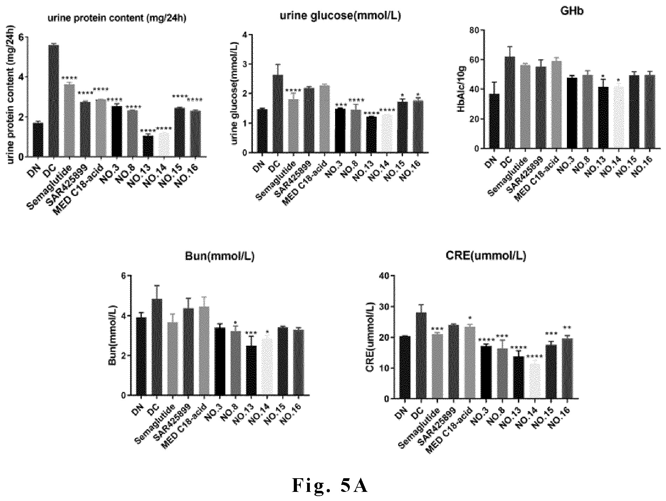

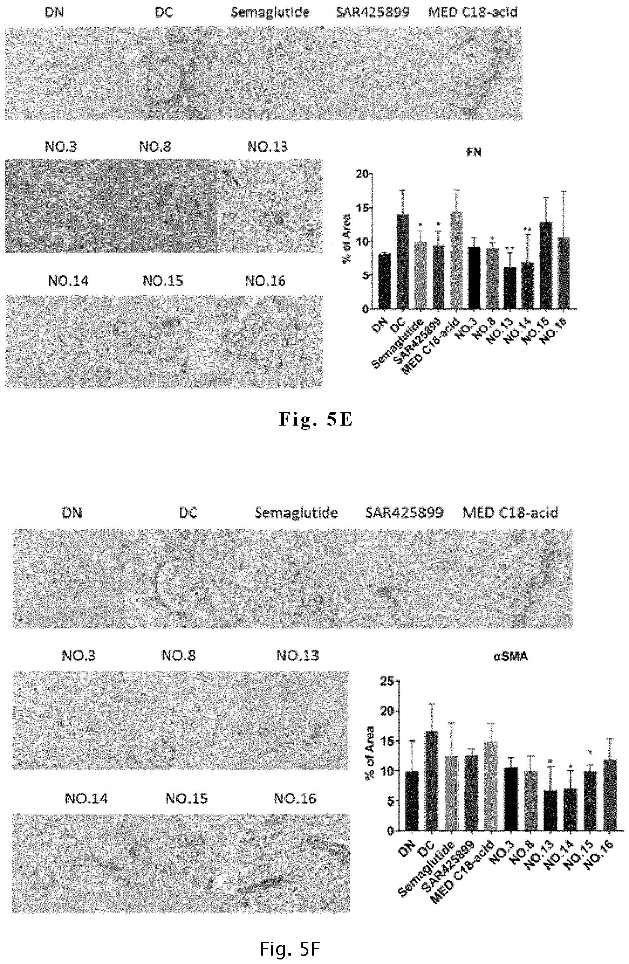

After 6 weeks of medical treatment, the mice was collected for urine using metabolic cages, and the protein content in the urine was detected using relevant kits. After 7 weeks of medical treatment, the material was taken and blood was used to detect serological indicators such as blood creatinine and urea nitrogen. The results are shown in Figs. 5A to 5F.

As shown in Fig. 5A, the 20-week-old db/db diabetic mice in the DC group showed a significant increase in urine protein content as well as urine glucose content compared to the control DN group. In addition, glycosylated hemoglobin content, blood creatinine, and urea nitrogen content were also significantly increased, indicating that the diabetic nephropathy in the mice was already severe. After the administration of the medicament, the urine protein content, urine glucose content, glycosylated hemoglobin content, blood creatinine and urea nitrogen content of the mice were all improved to different degrees.

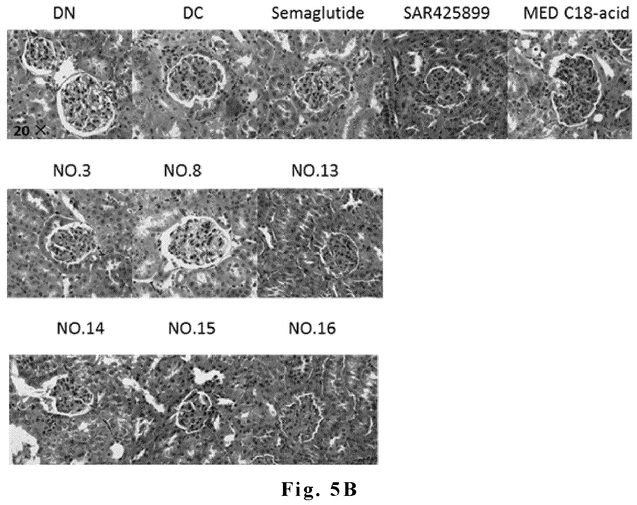

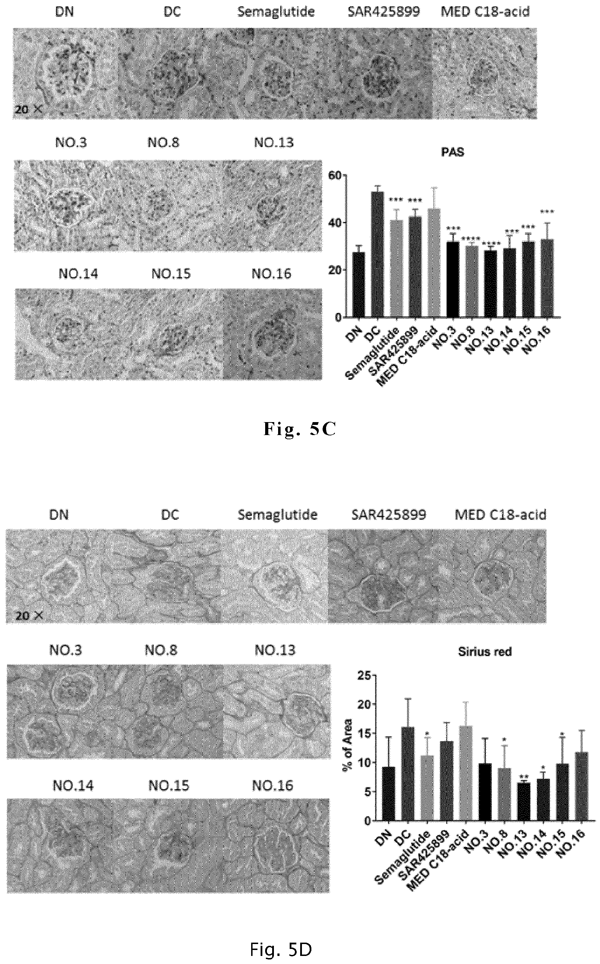

The kidneys of mice were taken for paraffin embedding and used for subsequent staining and immunohistochemical studies. According to the results of H&E staining of mouse kidneys in Fig. 5B, local inflammation and glomerular adhesions were observed in the kidney tissue. After administration, the glomerular adhesion phenomenon was alleviated, and the ameliorative and therapeutic effects of polypeptide compounds 3, 8, 13, 14, 15 and 16 were more significant compared to the positive control medicament semaglutide, SAR425899 and MED C18-acid groups. In general, renal lesions were observed mostly in the cortex, mainly including glomeruli and interstitium, and glomeruli were observed in the mesangial region. As observed by PAS staining (Fig. 5C), compared with the DN group, the glomerular mesangial region was widened, the mesangial cells and mesangial matrix were proliferated, and the basement membrane was thickened in the DC group. After administration of the medicament, the glomerular vascular collaterals were thin and clear, the number of endothelial and mesangial cells was normal, and the surrounding tubules were normal, and the ameliorative effects of polypeptide compounds 3, 8, 13, 14, 15 and 16 were more significant. As shown by the staining of Sirius novel red as well as FN and α-SMA in Figs. 5D to 5F, compared with the DN group, the collagen deposition was significantly increased in the DC group, and the medicament group could significantly reduce the collagen content, in which the polypeptide compounds 3, 8, 13, 14, 15 and 16 improved more significantly, and their therapeutic effect was better than the positive control semaglutide, SAR425899 and MED C18-acid groups.

In conclusion, by further optimization and design of the amino acid sequence, along with modification of the side chain technology, the compounds reported in the present invention are superior to the previously reported compounds in terms of hypoglycemia, weight reduction, improvement and treatment of diabetic complications, and are more advantageous in clinical applications.

The above description of specific embodiments of the present invention does not limit the present invention, and various changes or variations can be made by those skilled in the art according to the present invention, which shall fall within the scope of the appended claims of the present invention as long as they do not depart from the spirit of the present invention.

## Claims

1. A polypeptide compound comprising a parent peptide represented by the following amino acid sequence: 
His-Xaa2-Gln-Gly-Thr5-Phe-Thr-Ser-Asp-Lys10-Ser-Lys-Tyr-Leu-X
aa1515-Xaa16-Xaa17-Ala-Ala-Gln20-Xaa21-Phe-Xaa23-Xaa24-Trp25-Leu
-Xaa27-Xaa28-Gly-Gly30-Pro-Ser-Ser-Gly-Xaa3535-Pro-Pro-Pro-Ser,
 wherein,
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gln;
Xaa27 = Leu or Lys;
Xaa28 = Asp, Glu or Ala;
Xaa35 = Ala or Aib.

2. The polypeptide compound according to claim 1, wherein Xaa2 = Aib or D-Ser.

3. The polypeptide compound according to claim 2, wherein Xaa21 = Asp.

4. The polypeptide compound according to claim 3, wherein a carboxyl terminal of the amino acid sequence of the parent peptide is not modified or is modified by an amino group to form a -CONH₂ group.

5. The polypeptide compound according to any one of claims 1 to 4, wherein a side chain of Lys at position 10 or 12 in the amino acid sequence of the parent peptide is linked to a lipophilic substituent via a bridging group; the bridging group is one selected from (PEG)ₘ, (PEG)ₘ-γGlu, (PEG)ₘ-Asp, (Gly)ₓ-(Gly-Ser)_{y}-(Gly)_{z}-, (Gly)ₓ-(Gly-Ser)_{y}-(Gly)_{z}-γGlu and (Gly)ₓ-(Gly-Ser)_{y}-(Gly)_{z}-Asp; the linkage is a formation of an amide bond by a amino group of the Lys at position 10 or 12 with a carboxyl group of a glycine residue of the bridging group or a carboxyl group of a (PEG)ₘ terminal modification at one end of the bridging group, and the lipophilic substituent is attached to the amino group of the bridging group at the other end thereof by forming an amide bond with its carboxyl group; the lipophilic substituent is CH₃(CH₂)ₙC(O)- or HOOC(CH₂)ₙC(O)- and its acyl group forms an amide bond with the amino group in the bridging group; wherein m is an integer of 2 to 10; n is an integer of 14 to 20; x is an integer of 0 to 5; y is an integer of 1-5; z is an integer of 1-5.

6. The polypeptide compound according to claim 1, wherein the Lys at position 10 or 12 in the amino acid sequence of the parent peptide is replaced with HomoLys, Orn, Dap or Dab.

7. The polypeptide compound according to claim 1, wherein the parent peptide has in its amino acid sequence:
Xaa2 = Aib or D-Ser;
Xaa15 = Glu;
Xaa16 = Aib;
Xaa17 = Lys;
Xaa21 = Asp;
Xaa23 = Val;
Xaa24 = Glu;
Xaa27 = Lys;
Xaa28 = Ala;
Xaa35 = Ala.

8. The polypeptide compound according to claim 1, wherein the parent peptide has in its amino acid sequence:
Xaa2 = Aib or D-Ser;
Xaa15 = Asp;
Xaa16 = Glu;
Xaa17 = Arg;
Xaa21 = Asp;
Xaa23 = Iva;
Xaa24 = Gln;
Xaa27 = Leu;
Xaa28 = Asp or Glu;
Xaa35 = Ala or Aib.

9. The polypeptide compound according to claim 1, wherein the amino acid sequence of the parent peptide is selected from SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. ID NO. 10, SEQ ID NO. 11, SEQ ID NO. 12, SEQ ID NO. 13, SEQ ID NO. 14, SEQ ID NO. 15 and SEQ ID NO. 16.

10. The polypeptide compound according to claim 5, wherein the Lys at position 10 or 12 in the amino acid sequence of the parent peptide is linked via the bridging group to the lipophilic substituent to form any one of the following structures:
Lys(PEG₂-PEG₂-γGlu-CO(CH₂)₁₈CO₂H); 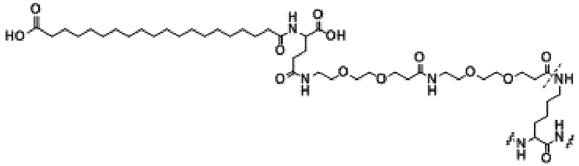 , or
Lys(PEG₂-PEG₂-γGlu-CO(CH₂)₁₈CO₃H); 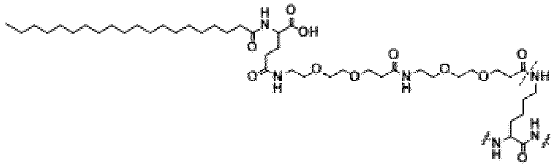 , or
Lys(PEG₂-PEG₂-γGlu-CO(CH₂)₁₅CO₂H); 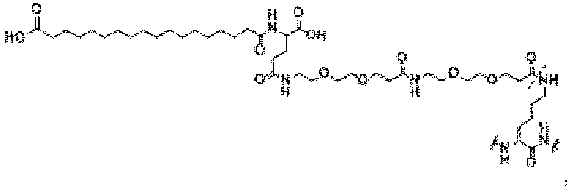 , or
Lys(PEG₂-PEG₂-CO(CH₂)₁₈CO₂H); 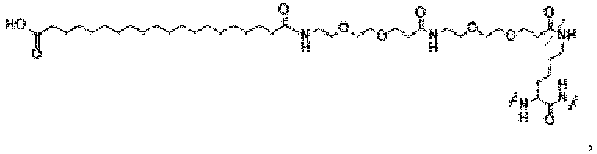 or
Lys(Gly-Gly-Ser-Gly-Ser-Gly-γGlu-CO(CH₂)₁₈CO₂H)

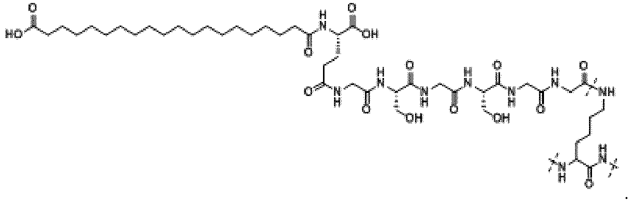

11. The polypeptide compound according to claim 1, wherein the polypeptide compound is any one of the following compounds:
Compound 1:
H-Aib-QGTFTSDK(PEG₂-PEG₂-γGlu-CO(CH₂)₁₈CO₂H)SKYLE-Ai b-KAAQDFVEWLKAGGPSSGAPPPS-NH₂
Compound 2:
HsQGTFTSDK(PEG₂-PEG₂-CO(CH₂)₁₈CO₂H)SKYLE-Aib-KAAQ DFVEWLKAGGPSSGAPPPS-NH₂
Compound 3:
HsQGTFTSDK(PEG₂-PEG₂-γGlu-CO(CH₂)₁₈CO₂H)SKYLE-Aib-K AAQDFVEWLKAGGPSSGAPPPS-NH₂
Compound 4:
HsQGTFTSDK(PEG₂-PEG₂-γGlu-CO(CH₂)₁₆CO₂H)SKYLE-Aib-K AAQDFVEWLKAGGPSSGAPPPS-NH₂
Compound 5:
HsQGTFTSDK(PEG₂-PEG₂-γGlu-CO(CH₂)₁₈CH₃)SKYLE-Aib-KA AQDFVEWLKAGGPSSGAPPPS-NH₂
Compound 6:
HsQGTFTSDK(PEG₂-PEG₂-γGlu-CO(CH₂)₁₈CO₂H)SKYLDERAA QDF-Iva-QWLLDGGPSSG-Aib-PPPS-NH₂
Compound 7:
HsQGTFTSDK(PEG₂-PEG₂-γGlu-CO(CH₂)₁₈CO₂H)SKYLDERAA QDF-Iva-QWLLEGGPSSGAPPPS-NH₂
Compound 8:
H-Aib-QGTFTSDK(PEG₂-PEG₂-γGlu-CO(CH₂)₁₈CO₂H)SKYLDER AAQDF-Iva-QWLLDGGPSSG-Aib-PPPS-NH₂
Compound 9:
H-Aib-QGTFTSDK(PEG₂-PEG₂-yGlu-CO(CH₂)₁₆CO₂H)SKYLDER AAQDF-Iva-QWLLDGGPSSG-Aib-PPPS-NH₂
Compound 10:
H-Aib-QGTFTSDK(PEG₂-PEG₂-γGlu-CO(CH₂)₁₈CH₃)SKYLDERA AQDF-Iva-QWLLDGGPSSG-Aib-PPPS-NH₂
Compound 11:
H-Aib-QGTFTSDK(PEG₂-PEG₂-γGlu-CO(CH₂)₁₈CH₃)SKYLDERA AQDF-Iva-QWLLDGGPSSG-Aib-PPPS-NH₂
Compound 12:
HsQGTFTSDK(PEG₂-PEG₂-γGlu-CO(CH₂)₁₈CO₂H)SKYLDERAA QDF-Iva-QWLLDGGPSSG-Aib-PPPS-NH₂
Compound 13:
HsQGTFTSDK(GGSGSG-γGlu-CO(CH₂)₁₈-COOH)SKYLE-Aib-K AAQDF VEWLKAGGPS SGAPPPS
Compound 14:
HsQGTFTSDK(GGSGSG-γGlu-CO(CH₂)₁₈-COOH)SKYLE-Aib-K AAQDFVEWLKAGGPSSGAPPPS-NH₂
Compound 15:
H-Aib-QGTFTSDK(GGSGSG-γGlu-CO(CH₂)₁₈-COOH)SKYLDER AAQDF-Iva-QWLLDGGPSSG-Aib-PPPS
Compound 16:
H-Aib-QGTFTSDK(GGSGSG-γGlu-CO(CH₂)₁₈-COOH)SKYLDER AAQDF-Iva-QWLLDGGPSSG-Aib-PPPS-NH₂ .

12. A composition comprising the polypeptide compound of any one of claims 1 to 11.

13. The composition according to claim 12, wherein the composition is a pharmaceutical composition, further comprising a pharmaceutically acceptable carriers or excipients.

14. Use of the polypeptide compound of any one of claims 1 to 11 or the composition of any one of claims 12 to 13 in the preparation of a medicament for preventing or treating diabetes and/or diabetic complications; and the diabetic complication is diabetic nephropathy.

15. Use of the polypeptide compound of any one of claims 1 to 11 or the composition of any one of claims 12 to 13 in the preparation of a nutraceutical or medicament for reducing body weight.
